# EUROPÄISCHE PATENTANMELDUNG

(11) **EP 1 800 655 A1**
(43) Veröffentlichungstag der Anmeldung: **27.06.2007**
(21) Anmeldenummer: 06022361.7
(22) Anmeldetag: 26.10.2006
(51) Int. Cl.: A61K 8/49, A61Q 5/10

(54) **Verwendung von Oniumaldehyden in Kombination mit Imidazolylalkyl-substituierten p-Phenylendiaminderivate**

(30) Priorität: 22.12.2005 DE 102005062013
(71) Anmelder: Henkel Kommanditgesellschaft auf Aktien, 40589 Düsseldorf (DE)
(72) Erfinder: Oberkobusch, Doris, 40591 Düsseldorf (DE); Höffkes, Horst, 40595 Düsseldorf (DE); Knübel, Georg, 40219 Düsseldorf (DE)

(57) **Zusammenfassung**

Färbemittel für keratinhaltige Fasern, insbesondere menschliche Haare, die Oniumaldehyde in Kombination mit mindestens einem Imidazolylalkyl-substituierten p-Phenylendiaminderivat enthalten, wirken nicht sensibilisierend, liefern intensive und äußerst gleichmäßige blaue bis violette Färbungen auf Fasern mit unterschiedlicher Faserstruktur mit einer hervorragenden Echtheit.

## Beschreibung

Die Erfindung betrifft Färbemittel für keratinhaltige Fasern, insbesondere menschliche Haare, die Oniumaldehyde in Kombination mit mindestens einem Imidazolylalkylsubstituierten p-Phenylendiaminderivat enthält, sowie ein Verfahren zum Färben von keratinhaltigen Fasern mit diesem Mittel.

Für das Färben von keratinhaltigen Fasern, z. B. Haaren, Wolle oder Pelzen, kommen im allgemeinen entweder direktziehende Farbstoffe oder Oxidationsfarbstoffe, die durch oxidative Kupplung einer oder mehrerer Entwicklerkomponenten untereinander oder mit einer oder mehreren Kupplerkomponenten entstehen, zur Anwendung. Kuppler- und Entwicklerkomponenten werden auch als Oxidationsfarbstoffvorprodukte bezeichnet.

Als Entwicklerkomponenten werden üblicherweise primäre aromatische Amine mit einer weiteren, in para- oder ortho-Position befindlichen freien oder substituierten Hydroxy-oder Aminogruppe, Diaminopyridinderivate, heterocyclische Hydrazone, 4-Aminopyrazolonderivate sowie 2,4,5,6-Tetraaminopyrimidin und dessen Derivate eingesetzt.

Spezielle Vertreter sind beispielsweise p-Phenylendiamin, p-Toluylendiamin, 2,4,5,6-Tetraaminopyrimidin, p-Aminophenol, N,N-Bis-(2-hydroxyethyl)-p-phenylendiamin, 2-(2,5-Diaminophenyl)-ethanol, 2-(2,5-Diaminophenoxy)-ethanol, 1-Phenyl-3-carboxyamido-4-amino-pyrazolon-5, 4-Amino-3-methylphenol, 2-Aminomethyl-4-aminophenol, 2-Hydroxymethyl-4-aminophenol, 2-Hydroxy-4,5,6-triaminopyrimidin, 2,4-Dihydroxy-5,6-diaminopyrimidin und 2,5,6-Triamino-4-hydroxypyrimidin.

Als Kupplerkomponenten werden in der Regel m-Phenylendiaminderivate, Naphthole, Resorcin und Resorcinderivate, Pyrazolone und m-Aminophenole verwendet. Als Kupplersubstanzen eignen sich insbesondere α-Naphthol, 1,5-, 2,7- und 1,7-Dihydroxynaphthalin, 5-Amino-2-methylphenol, m-Aminophenol, Resorcin, Resorcinmonomethylether, m-Phenylendiamin, 2,4-Diaminophenoxyethanol, 1-Phenyl-3-methyl-pyrazolon-5, 2,4-Dichlor-3-aminophenol, 1,3-Bis-(2,4-diaminophenoxy)-propan, 2-Chlorresorcin, 4-Chlorresorcin, 2-Chlor-6-methyl-3-aminophenol, 2-Methylresorcin und 5-Methylresorcin.

Bezüglich weiterer üblicher Farbstoffkomponenten wird ausdrücklich auf die Reihe "Dermatology", herausgeben von Ch. Culnan, H. Maibach, Verlag Marcel Dekker Inc., New York, Basel, 1986, Bd. 7, Ch. Zviak, The Science of Hair Care, Kap. 7, Seiten 248 - 250 (Direktziehende Farbstoffe) und Kap. 8, Seiten 264 - 267 (Oxidationsfarbstoffe) sowie das "Europäische Inventar der Kosmetikrohstoffe", 1996, herausgegeben von der Europäischen Kommission, erhältlich in Diskettenform vom Bundesverband der deutschen Industrie- und Handelsunternehmen für Arzneimittel, Reformwaren und Körperpflegemittel e.V., Mannheim, Bezug genommen.

Mit Oxidationsfarbstoffen lassen sich zwar intensive Färbungen mit guten Echtheitseigenschaften erzielen, die Entwicklung der Farbe geschieht jedoch im Allgemeinen unter Zusatz chemischer Oxidationsmittel wie z.B. H₂O₂, was in einigen Fällen Schädigungen der Faser zur Folge haben kann. Desweiteren können einige Oxidationsfarbstoffvorprodukte bzw. bestimmte Mischungen von Oxidationsfarbstoffvorprodukten bisweilen bei Personen mit empfindlicher Haut sensibilisierend wirken. Direktziehende Farbstoffe werden unter schonenderen Bedingungen appliziert, ihr Nachteil liegt jedoch darin, daß die Färbungen häufig nur über unzureichende Echtheitseigenschaften verfügen.

Eine Möglichkeit, Färbungen mit guten Echtheitseigenschaften ohne den Einfluß von zusätzlichen chemischen Oxidationsmitteln zu erzielen ist die Anwendung von sogenannten Oxofärbemitteln. Oxofärbemittel bieten die Möglichkeit keratinhaltige Fasern zu färben, mittels Verwendung einer Kombination aus
Komponente A): Verbindungen, die eine reaktive Carbonylgruppe enthalten, mit
Komponente B): Verbindungen, ausgewählt aus (a) CH-aciden Verbindungen, (b) Verbindungen mit primärer oder sekundärer Aminogruppe, ausgewählt aus primären oder sekundären aromatischen Aminen, stickstoffhaltigen heterozyklischen Verbindungen und aromatischen Hydroxyverbindungen, (c) Aminosäuren, (d) aus 2 bis 9 Aminosäuren aufgebauten Oligopeptiden enthalten.

Die entsprechende Färbemethode (im folgenden Oxofärbung genannt) wird beispielsweise in den Druckschriften WO-A1-99/18916, WO-A1-00/38638, WO-A1-01/34106 und WO-A1-01/47483 beschrieben. Die resultierenden Färbungen besitzen teilweise Farbechtheiten auf der keratinhaltigen Faser, die mit denen der Oxidationsfärbung vergleichbar sind. Das mit der schonenden Oxofärbung erzielbare Nuancenspektrum ist sehr breit und die erhaltene Färbung weist oftmals eine akzeptable Brillanz und Farbtiefe auf. Die vorgenannten Komponenten A und B, im weiteren als Oxofarbstoffvorprodukte bezeichnet, sind im allgemeinen selbst keine Farbstoffe, und eignen sich daher jede für sich genommen allein nicht zur Färbung keratinhaltiger Fasern. In Kombination bilden sie in einem nichtoxidativen Prozess Farbstoffe aus. Unter Verbindungen der Komponente B können allerdings auch entsprechende Oxidationsfarbstoffvorprodukte vom Entwickler- und/oder Kupplertyp mit oder ohne Einsatz eines Oxidationsmittels Verwendung finden. Somit läßt sich die Methode der Oxofärbung ohne weiteres mit dem oxidativen Färbesystem kombinieren.

Im Rahmen der Oxofärbung werden als Komponente A reaktive Carbonylverbindungen eingesetzt, die insbesondere nach Reaktion mit einer Komponente B, den eigentlichen Farbstoff im Haar ausbilden. Bevorzugte reaktive Carbonylverbindungen sind Aldehyde und Ketone, in denen die reaktive Carbonylgruppe entweder als Carbonylgruppe vorliegt oder derart derivatisiert bzw. maskiert ist, daß die Reaktivität des Kohlenstoffatoms der derivatisierten Carbonylgruppe gegenüber den Verbindungen der Komponente B stets vorhanden ist. Diese Derivate sind bevorzugt Additionsverbindungen
a) von Aminen und deren Derivaten unter Bildung von Iminen oder Oximen als Additionsverbindung
b) von Alkoholen unter Bildung von Acetalen oder Ketalen als Additionsverbindung
c) von Wasser unter Bildung von Hydraten als Additionsverbindung (Komponente A leitet sich in diesem Fall c) von einem Aldehyd ab)
   an das Kohlenstoffatom der Carbonylgruppe der reaktiven Carbonylverbindung.

Als hervorragende reaktive Carbonylverbindungen der Komponente A) haben sich Oniumaldehyde erwiesen, welche durch Reaktion mit den Verbindungen der Komponente B) intensive Färbungen erzielen. Wichtige Kriterien für die erfolgreiche gewerbliche Nutzung von Färbemitteln sind die Egalisierung des Farbaufzugs, eine geringe bis nicht vorhandene Hautanfärbung, die Intensität der Färbung, die Echtheit der Färbung gegenüber Umwelteinflüssen und nicht zuletzt die physiologische Verträglichkeit des Färbemittels.

Bisher fehlt es an Farbstoffkombinationen für eine Blau bis Blauviolettfärbung mit optimalem Färbeverhalten und zugleich guter physiologischer Verträglichkeit. Das Färbeverhalten soll insbesondere in den Parametern der Farbechtheit (gegenüber Waschen, Licht, Reibung, Schweiß und Kaltwellmitteln), der minimalen bis keinen Anfärbung der Haut sowie der Gleichmäßigkeit der Ausfärbung auf unterschiedlich strukturierten keratinhaltigen Fasern optimiert sein. Das Phänomen der unterschiedlich strukturierten keratinhaltigen Fasern tritt verstärkt bei natürlichen Fasern, insbesondere beim Haar, auf, wenn der Haarwuchs Haare oder Haarzonen unterschiedlichen Schädigungsgrades aufweist. Ein Beispiel dafür sind lange Haare, bei denen die Haarspitzen über einen längeren Zeitraum den verschiedensten Umwelteinflüssen ausgesetzt sind und daher in der Regel deutlich stärker geschädigt sind als die relativ frisch nachgewachsenen Haarzonen am Haaransatz.

Aufgabe der vorliegenden Erfindung ist es, Oxofärbemittel für keratinhaltige Fasern, insbesondere menschliche Haare, bereitzustellen, die einen Blau- bis Blauviolettfarbton besitzen, ein geringes Sensibilisierungspotenzial besitzen, über die gesamte Länge der Faser gleichmäßig intensive Färbungen liefern und die Haut weniger anfärben. Darüber hinaus sollten die Färbungen verbesserte Farbechtheiten aufweisen.

Unter keratinhaltigen Fasern sind Wolle, Pelze, Federn und insbesondere menschliche Haare zu verstehen. Die erfindungsgemäßen Färbemittel können prinzipiell aber auch zum Färben anderer Naturfasern, wie z.B. Baumwolle, Jute, Sisal, Leinen oder Seide, modifizierter Naturfasern, wie z.B. Regeneratcellulose, Nitro-, Alkyl- oder Hydroxyalkyl-oder Acetylcellulose und synthetischer Fasern, wie z.B. Polyamid-, Polyacrylnitril-, Polyurethan- und Polyesterfasern verwendet werden.

Es wurde überraschenderweise gefunden, dass diese Aufgabe durch die erfindungsgemäße Oxofarbstoffvorprodukt-Kombination gelöst wird.

Gegenstand der vorliegenden Erfindung ist demgemäß ein Mittel zum Färben keratinhaltiger Fasern, insbesondere menschlicher Haare, welches eine Kombination aus Komponente
(A) mindestens einer Verbindung der Formel I oder deren Derivaten 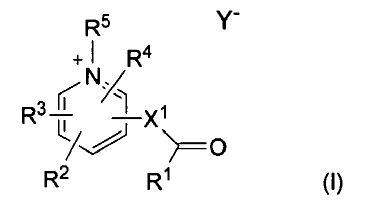 worin
   R¹ für ein Wasserstoffatom, eine (C₁-C₆)-Alkylgruppe, eine Arylgruppe oder eine Heteroarylgruppe steht,
   R², R³ und R⁴ jeweils unabhängig voneinander ein Wasserstoffatom, ein Halogenatom, eine (C₁-C₆)-Alkylgruppe, (C₁-C₆)-Alkoxygruppe, (C₁-C₆)-Hydroxylalkylgruppe, Hydroxy-(C₁-C₆)-alkoxygruppe, Hydroxygruppe, Nitrogruppe, Arylgruppe, Trifluormethylgruppe, Aminogruppe, die durch (C₁-C₆)-Alkylgruppen substituiert sein kann, oder (C₁-C₆)-Acylgruppe bedeuten, wobei auch zwei der Reste gemeinsam einen ankondensierten Benzolring bilden können,
   R⁵ für eine (C₁-C₆)-Alkylgruppe, Arylgruppe, Aryl-(C₁-C₆)-alkylgruppe oder Heteroarylgruppe steht,
   X¹ für eine direkte Bindung oder eine ggf. substituierte Vinylen- oder Phenylengruppe steht, und
   Y⁻ ein physiologisch verträgliches Anion bedeutet,
   und Komponente
(B) mindestens einer Verbindung gemäß Formel (II), 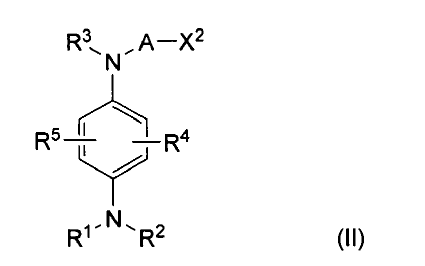
worin
- A steht für eine verzweigte oder unverzweigte Alkylengruppe mit 1 bis 6 Kohlenstoffatomen, die gegebenenfalls einen oder mehrere Substituenten, ausgewählt aus Hydroxygruppe(n) und Halogenatom(en), tragen kann,
- X² steht für einen gegebenenfalls substituierten Imidazolylrest,
- R¹, R² und R³ stehen unabhängig voneinander für ein Wasserstoffatom, eine C₁- bis C₄-Alkylgruppe, eine C₁- bis C₄-Monohydroxyalkylgruppe oder eine C₂- bis C₆- Polyhydroxyalkylgruppe, und
- R⁴ und R⁵ stehen unabhängig voneinander für ein Wasserstoffatom, eine C₁- bis C₄-Alkylgruppe, eine C₁- bis C₄-Alkoxygruppe, eine C₁- bis C₄-Monohydroxyalkylgruppe, eine C₂- bis C₄- Polyhydroxyalkylgruppe oder ein Halogenatom,
enthält.

Unter Derivaten der Verbindungen gemäß Formel I sind im Sinne der Erfindung Additionsverbindungen
a) von Aminen und deren Derivaten unter Bildung von Iminen oder Oximen als Additionsverbindung
b) von Alkoholen unter Bildung von Acetalen oder Ketalen als Additionsverbindung
c) von Wasser unter Bildung von Hydraten als Additionsverbindung (Komponente A leitet sich in diesem Fall c) von einem Aldehyd ab)
   an das Kohlenstoffatom der Carbonylgruppe der reaktiven Carbonylverbindung zu verstehen. Insbesondere sind Hydrate als Derivate gemäß c) bevorzugt.

Beispiele für die als Substituenten im Rahmen dieser Anmeldung genannten (C₁-C₆)-Alkylreste sind die Gruppen Methyl, Ethyl, Propyl, Isopropyl, n-Butyl, tert-Butyl, n-Pentyl, neo-Pentyl und n-Hexyl. Ethyl und Methyl sind bevorzugte Alkylreste. Beispiele für bevorzugte (C₂-C₆)-Alkenylreste sind Vinyl, Allyl und Butenyl. Erfindungsgemäß bevorzugte (C₁-C₆)-Alkoxyreste sind beispielsweise eine Methoxy- oder eine Ethoxygruppe. Weiterhin können als bevorzugte Beispiele für eine (C₁-C₆)-Hydroxyalkylgruppe eine Hydroxymethyl-, eine 2-Hydroxyethyl-, eine 2-Hydroxypropyl, eine 3-Hydroxypropyl- oder eine 4-Hydroxybutylgruppe genannt werden. Eine 2-Hydroxyethylgruppe ist besonders bevorzugt. Beispiele für eine bevorzugte (C₂-C₆)-Polyhydroxyalkylgruppe sind die 1,2-Dihydroxyethylgruppe und die 2,3-Dihydroxypropylgruppe. Beispiele für eine (C₁-C₆)-Acylgruppe sind Acetyl, Propanoyl und Butanoyl. Bevorzugte Hydroxy-(C₁-C₆)-alkoxygruppen sind 2-Hydroxyethoxy, 2-Hydroxypropoxy und 3-Hydroxypropoxy. Bevorzugte Arylgruppen sind Phenyl, Naphthyl und Biphenyl. Beispiele für eine Heteroarylgruppe sind Pyrrolidyl, 2-Furyl, 2-Thienyl, 4-Pyridyl, 3-Pyridyl, 2-Pyridyl, Triazolyl und 1-Imidazolyl. Bevorzugte Aryl-(C₁-C₆)-alkylgruppen sind Benzyl, 2-Phenylethyl, 3-Phenylpropyl, wobei Benzyl besonders bevorzugt ist. Beispiele für Halogenatome sind F-, Cl-, oder Br-Atome, wobei Cl-Atome ganz besonders bevorzugt sind. Die weiteren verwendeten Begriffe leiten sich erfindungsgemäß von den hier gegebenen Definitionen ab.

Y⁻ steht gemäß Formel I bevorzugt für Halogenid, Benzolsulfonat, p-Toluolsulfonat, Methansulfonat, Trifluormethansulfonat, Perchlorat, Sulfat, Hydrogensulfat, Tetrachlorzinkat oder N-Oxid des Chinolins, besonders bevorzugt für ein p-Toluolsulfonat-, Methansulfonat-, Trifluormethansulfonat-, Methylsulfat- oder Benzolsulfonat-Ion. Das bevorzugte Anion ist das p-Toluolsulfonat-Ion.

Die Verbindungen mit der Formel I werden üblicherweise in einer Menge von 0,03 bis 65 mmol, insbesondere 1 bis 40 mmol, jeweils bezogen auf 100 g des anwendungsbereiten Mittels, eingesetzt.

Es ist erfindungsgemäß bevorzugt, wenn die Verbindungen der Formel (I) aus Verbindungen der Formel (III) ausgewählt werden, 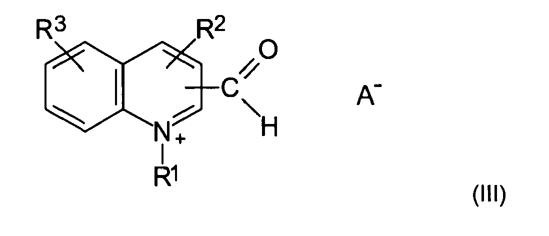 worin
- R¹: für eine (C₁-C₆)-Alkylgruppe, (C₂-C₆)-Alkenylgruppe, Arylgruppe, Aryl-(C₁-C₆)-alkylgruppe oder Heteroaryl-(C₁-C₆)-alkylgruppe steht,
- R² und R³: jeweils unabhängig voneinander ein Wasserstoffatom, ein Halogenatom, eine (C₁-C₆)-Alkylgruppe, eine (C₁-C₆)-Alkoxygruppe, Hydroxy-(C₁-C₆)-alkoxygruppe, Hydroxygruppe, Nitrogruppe, Arylgruppe, Trifluormethylgruppe, Aminogruppe, die durch (C₁-C₆)-Alkylgruppen substituiert sein kann, oder (C₁-C₆)-Acylgruppe bedeuten und
- A⁻: ein physiologisch verträgliches Anion bedeutet.

Bevorzugte Verbindungen gemäß Formel III sind solche, worin die Formylgruppe -CHO in 2- oder 4-Position gebunden ist.

A⁻ steht gemäß Formel III bevorzugt für Halogenid, Benzolsulfonat, p-Toluolsulfonat, Methansulfonat, Trifluormethansulfonat, Perchlorat, Sulfat, Hydrogensulfat, Tetrachlorzinkat oder N-Oxid des Chinolins, besonders bevorzugt für ein p-Toluolsulfonat-, Methansulfonat-, Trifluormethansulfonat-, Methylsulfat- oder Benzolsulfonat-lon. Das bevorzugte Anion ist das p-Toluolsulfonat-lon.

R¹ steht gemäß Formel III bevorzugt für eine (C₁-C₆)-Alkylgruppe.

Die Verbindungen gemäß Formel III werden besonders bevorzugt ausgewählt aus der Gruppe bestehend aus den Benzolsulfonaten, p-Toluolsulfonaten, Methansulfonaten, Trifluormethansulfonaten, Perchloraten, Sulfaten, Chloriden, Bromiden, Jodiden und/oder Tetrachlorzinkaten von 4-Formyl-1-methylchinolinium und 2-Formyl-1-methylchinolinium. 4-Formyl-1-methyl-chinolinium p-Toluolsulfonat und 2-Formyl-1-methyl-chinolinium p-Toluolsulfonat sind ganz besonders bevorzugte Verbindungen gemäß Formel I.

Da es sich bei den erfindungsgemäßen Verbindungen mit der Formel (II) um AminoVerbindungen handelt, lassen sich aus diesen in üblicher Weise die bekannten Salze als Säureadditionssalze herstellen. Alle Aussagen dieser Schrift und demgemäß der beanspruchte Schutzbereich beziehen sich daher sowohl auf die in freier Form vorliegenden Verbindungen als auch auf deren wasserlösliche, physiologisch verträgliche Salze. Beispiele für solche Salze sind die Hydrochloride, die Hydrobromide, die Sulfate, die Phosphate, die Acetate, die Propionate, die Citrate und die Lactate. Die Hydrochloride und die Sulfate sind dabei besonders bevorzugt.

Ein wesentliches Merkmal der erfindungsgemäßen Verbindungen der Formel (II) ist der gegebenenfalls substituierte Imidazolylrest. In einer ersten bevorzugten Ausführungsform der vorliegenden Erfindung, steht die Gruppe X² für einen gegebenenfalls substituierten Imidazolylrest der Formel (IVa) 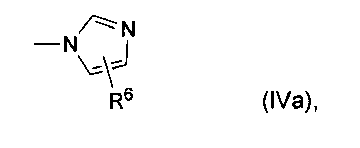 wobei R⁶ steht für ein Wasserstoffatom, eine C₁- bis C₄-Alkylgruppe, eine C₁- bis C₄-Monohydroxyalkylgruppe, eine C₂- bis C₄- Polyhydroxyalkylgruppe oder ein Halogenatom.

Im Rahmen dieser Ausführungsform sind Verbindungen der Formel (II) besonders bevorzugt, bei denen A steht für eine unverzweigte Alkylengruppe mit 2 bis 6 Kohlenstoffatomen. Eine besonders bevorzugte Alkylengruppe ist im Rahmen dieser Ausführungsform die Ethan-1,2-diylgruppe und die Propan-1,3-diylgruppe.

Ganz besonders bevorzugte Verbindungen der Formel (II) mit einem Imidazolylrest der Formel (IVa) sind zum einen das (4-Aminophenyl)(3-(imidazol-1-yl)propyl)amin der Formel (V-1) 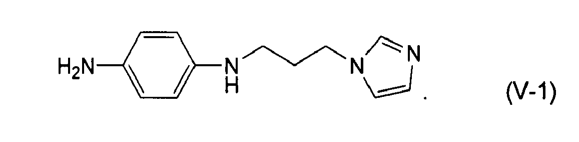 und zum anderen das (4-Amino-3-methylphenyl)(3-(imidazol-1-yl)propyl)amin der Formel (V-2) 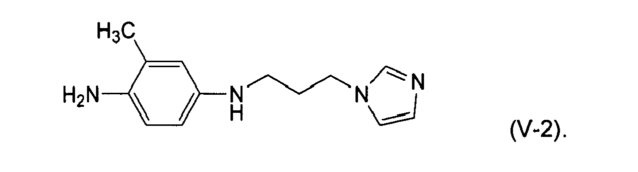 Im Rahmen einer zweiten bevorzugten Ausführungsform der vorliegenden Erfindung handelt es sich bei dem gegebenenfalls substituierten Imidazolylrest X² der Formel (II) um eine Gruppe der Formel (IVb) 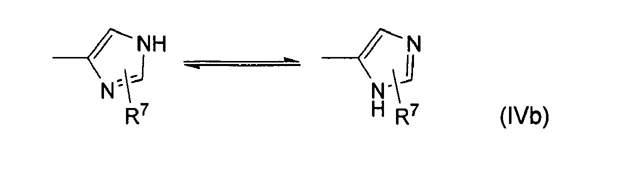 wobei R⁷ steht für ein Wasserstoffatom, eine C₁- bis C₄-Alkylgruppe, eine C₁- bis C₄-Monohydroxyalkylgruppe, eine C₂- bis C₄- Polyhydroxyalkylgruppe oder ein Halogenatom. Da die Verbindungen dieser Ausführungsform der vorliegenden Erfindung in Form eines tautomeren Gleichgewichtes vorliegen, beziehen sich die Aussagen dieser Schrift auf beide im Gleichgewicht vorliegenden Tautomere.

Im Rahmen dieser Ausführungsform der vorliegenden Erfindung steht die Gruppe A bevorzugt für eine unverzweigte Alkylengruppe mit 1 bis 6 Kohlenstoffatomen. Besonders bevorzugter Weise ist die Gruppe A eine Ethylengruppe.

Eine ganz besonders bevorzugte Verbindung der Formel (II) mit einem Imidazolylrest der Formel (IVb) ist (4-Aminophenyl)(2-(imidazol-5-yl)ethyl)amin der Formel (VI)

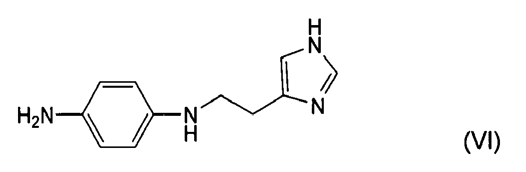

Die Verbindungen der Formel (II) sind in den erfindungsgemäßen Mitteln bevorzugt in einer Menge von 0,03 bis 65 mmol, besonders bevorzugt von 1 bis 40 mmol, jeweils bezogen auf das 100 g des anwendungsbereiten Mittels, enthalten.

Als optionale dritte Komponente C können die erfindungsgemäßen Mittel zusätzlich mindestens eine Verbindung, ausgewählt aus CH-aciden Verbindungen und/oder Verbindungen mit primärer oder sekundärer Aminogruppe, ausgewählt aus der Gruppe, die gebildet wird aus primären oder sekundären aromatischen Aminen, stickstoffhaltigen heterocyclischen Verbindungen und aromatischen Hydroxyverbindungen. Diese Verbindungen sind von den Verbindungen der Formel (II) verschieden.

Als CH-acide werden im allgemeinen solche Verbindungen angesehen, die ein an ein aliphatisches Kohlenstoffatom gebundenes Wasserstoffatom tragen, wobei durch eine Aktivierung der entsprechenden Kohlenstoff-Wasserstoff-Bindung dieses Wasserstoffatom mit einer Base abstrahiert werden kann.

Die CH-aciden Verbindungen sind bevorzugt ausgewählt aus der Gruppe bestehend aus 1,2,3,3-Tetramethyl-3H-indoliumiodid, 1,2,3,3-Tetramethyl-3H-indolium-p-toluolsulfonat, 1,2,3,3-Tetramethyl-3H-indolium-methansulfonat, 1,3,3-Trimethyl-2-methylenindolin (Fischersche Base), 2,3-Dimethyl-benzothiazoliumiodid, 2,3-Dimethyl-benzothiazoliump-toluolsulfonat, 2,3-Dimethyl-naphtho[1,2-d]thiazolium-p-toluolsulfonat, 3-Ethyl-2-methyl-naphtho[1,2-d]thiazolium-p-toluolsulfonat, Rhodanin, Rhodanin-3-essigsäure, 1,4-Dimethylchinolinium-iodid, 1,2-Dimethylchinolinium-iodid, Barbitursäure, Thiobarbitursäure, 1,3-Dimethylthiobarbitursäure, 1,3-Diethylthiobarbitursäure, 1,3-Diethylbarbitursäure, Oxindol, 3-Indoxylacetat, 2-Cumaranon, 5-Hydroxy-2-cumaranon, 6-Hydroxy-2-cumaranon, 3-Methyl-1-phenyl-pyrazolin-5-on, Indan-1,2-dion, Indan-1,3-dion, Indan-1-on, Benzoylacetonitril, 3-Dicyanmethylenindan-1-on, 2-Amino-4-imino-1,3-thiazolin-hydrochlorid, 5,5-Dimethylcyclohexan-1,3-dion, 2H-1,4-Benzoxazin-4H-3-on, 3-Ethyl-2-methyl-benzoxazoliumiodid, 3-Ethyl-2-methyl-benzothiazoliumiodid, 1-Ethyl-4-methyl-chinoliniumiodid, 1-Ethyl-2-methylchinoliniumiodid, 1,2,3-Trimethylchinoxaliniumiodid, 3-Ethyl-2-methyl-benzoxazolium-p-toluolsulfonat, 3-Ethyl-2-methyl-benzothiazolium-p-toluolsulfonat, 1-Ethyl-4-methyl-chinolinium-p-toluolsulfonat, 1-Ethyl-2-methylchinolinium-p-toluolsulfonat, 1,2,3-Trimethylchinoxalinium-p-toluolsulfonat 1,2-Dihydro-1,3,4,6-tetramethyl-2-oxo-pyrimidinium-chlorid, 1,2-Dihydro-1,3-diethyl-4,6-dimethyl-2-oxo-pyrimidinium-chlorid, 1,2-Dihydro-1,3-dipropyl-4,6-dimethyl-2-oxopyrimidinium-chlorid, 1,2-Dihydro-1,3,4,6-tetramethyl-2-oxo-pyrimidinium-hydrogensulfat, 1,2-Dihydro-1,3-diethyl-4,6-dimethyl-2-oxo-pyrimidinium-hydrogensulfat, 1,2-Dihydro-1,3-dipropyl-4,6-dimethyl-2-oxo-pyrimidinium-hydrogensulfat, 1,2-Dihydro-1,3,4-trimethyl-2-oxo-pyrimidinium-chlorid, 1,2-Dihydro-1,3,4-trimethyl-2-oxo-pyrimidinium-hydrogensulfat, 1,2-Dihydro-1,3-diethyl-4-methyl-2-oxo-pyrimidinium-chlorid, 1,2-Dihydro-1,3-diethyl-4-methyl-2-oxo-pyrimidinium-hydrogensulfat, 1,2-Dihydro-1,3-dipropyl-4-methyl-2-oxo-pyrimidinium-chlorid, 1,2-Dihydro-1,3-dipropyl-4-methyl-2-oxo-pyrimidinium-hydrogensulfat, 1,2-Dihydro-1,3,4,6-tetramethyl-2-thioxo-pyrimidinium-chlorid, 1,2-Dihydro-1,3-diethyl-4,6-dimethyl-2-thioxo-pyrimidinium-chlorid, 1,2-Dihydro-1,3-dipropyl-4,6-dimethyl-2-thioxo-pyrimidinium-chlorid, 1,2-Dihydro-1,3,4,6-tetramethyl-2-thioxo-pyrimidinium-hydrogensulfat, 1,2-Dihydro-1,3-dipropyl-4,6-dimethyl-2-thioxo-pyrimidinium-hydrogensulfat, 1,2-Dihydro-1,3,4-trimethyl-2-thioxo-pyrimidinium-chlorid, 1,2-Dihydro-1,3,4-trimethyl-2-thioxo-pyrimidinium-hydrogensulfat, 1,2-Dihydro-1,3-diethyl-4-methyl-2-thioxo-pyrimidinium-chlorid, 1,2-Dihydro-1,3-diethyl-4-methyl-2-thioxo-pyrimidinium-hydrogensulfat, 1,2-Dihydro-1,3-dipropyl-4-methyl-2-thioxo-pyrimidinium-chlorid und 1,2-Dihydro-1,3-dipropyl-4-methyl-2-thioxo-pyrimidinium-hydrogensulfat.

Die primären und sekundären aromatischen Amine werden besonders bevorzugt ausgewählt aus der Gruppe bestehend aus N,N-Dimethyl-p-phenylendiamin, N,N-Diethyl-p-phenylendiamin, N-(2-Hydroxyethyl)-N-ethyl-p-phenylendiamin, N,N-Bis-(2-hydroxyethyl)-p-phenylendiamin, N-(2-Methoxyethyl)-p-phenylendiamin, 2,3-Dichlor-p-phenylendiamin, 2,4-Dichlor-p-phenylendiamin, 2,5-Dichlor-p-phenylendiamin, 2-Chlor-p-phenylendiamin, 2,5-Dihydroxy-4-morpholinoanilin, 2-Aminophenol, 3-Aminophenol, 4-Aminophenol, 2-Aminomethyl-4-aminophenol, 2-Hydroxymethyl-4-aminophenol, o-Phenylendiamin, m-Phenylendiamin, p-Phenylendiamin, 2,5-Diaminotoluol, 2,5,-Diaminophenol, 2,5-Diaminoanisol, 2,5,Diaminophenethof, 4-Amino-3-methylphenol, 2-(2,5-Diaminophenyl)-ethanol, 2-(2,4-Diaminophenoxy)ethanol, 2-(2,5-Diaminophenoxy)-ethanol, 3-Amino-4-(2-hydroxyethyloxy)phenol, 3,4-Methylendioxyphenol, 3,4-Methylendioxyanilin, 3-Amino-2,4-dichlorphenol, 4-Amino-2,6-dichlorphenol, 4-Methylaminophenol, 4-Aminochinaldin, 5-Amino-2-methylphenol, 4-Amino-3-methylphenol, 2-Methyl-5-(2-hydroxyethylamino)phenol, 3-Amino-2-chlor-6-methylphenol, 2-Amino-6-chlor-4-nitrophenol, 2-Methyl-5-amino-4-chlorphenol, 5-(2-Hydroxyethylamino)-4-methoxy-2-methylphenol, 4-Amino-2-hydroxymethylphenol, 2-(Diethylaminomethyl)-4-aminophenol, 4-Amino-1-hydroxy-2-(2-hydroxyethylaminomethyl)-benzol, 1-Hydroxy-2-amino-5-methylbenzol, 1-Hydroxy-2-amino-6-methyl-benzol, 2-Amino-5-acetamido-phenol, 1,3-Dimethyl-2,5-diaminobenzol, 5-(3-Hydroxypropylamino-)2-methylphenol, 5-Amino-4-methoxy-2-methylphenol, N,N-Dimethyl-3-aminophenol, N-Cyclopentyl-3-aminophenol, 5,6-Dihydroxyindolin, 4-Hydroxyindolin, 5-Amino-4-fluor-2-methylphenol, 2,4-Diamino-5-fluortoluol, 2,4-Diamino-5-(2-hydroxyethoxy)-toluol, 2,4-Diamino-5-methylphenetol, 3,5-Diamino-2-methoxy-1-methylbenzol, 2-Amino-4-(2-hydroxyethylamino)-anisol, 2,6-Bis-(2-hydroxyethylamino)-1-methylbenzol, 1,3-Diamino-2,4-dimethoxybenzol, 3,5-Diamino-2-methoxy-toluol, 2-Aminobenzoesäure, 3-Aminobenzoesäure, 4-Aminobenzoesäure, 2-Aminophenylessigsäure, 3-Aminophenylessigsäure, 4-Aminophenylessigsäure, 2,3-Diaminobenzoesäure, 2,4-Diaminobenzoesäure, 2,5-Diaminobenzoesäure, 3,4-Diaminobenzoesäure 3,5-Diaminobenzoesäure, 4-Aminosalicylsäure, 5-Aminosalicylsäure, 3-Amino-4-hydroxy-benzoesäure, 4-Amino-3-hydroxy-benzoesäure, 2-Aminobenzolsulfonsäure, 3-Aminobenzolsulfonsäure, 4-Aminobenzolsulfonsäure, 3-Amino-4-hydroxybenzolsulfonsäure, 4-Amino-3-hydroxynaphthalin-1-sulfonsäure, 6-Amino-7-hydroxynaphthalin-2-sulfonsäure, 7-Amino-4-hydroxynaphthalin-2-sulfonsäure, 4-Amino-5-hydroxynaphthalin-2,7-disulfonsäure, 3-Amino-2-naphthoesäure, 3-Aminophthalsäure, 5-Aminoisophthalsäure, 1,3,5-Triaminobenzol, 1,2,4-Triaminobenzol, 1,2,4,5-Tetraaminobenzol, 2,4,5-Triaminophenol, Pentaaminobenzol, Hexaaminobenzol, 2,4,6-Triaminoresorcin, 4,5-Diaminobrenzcatechin, 4,6-Diaminopyrogallol, 1-(2-Hydroxy-5-aminobenzyl)-2-imidazolidinon, 4-Amino-2-((4-[(5-amino-2-hydroxyphenyl)methyl]-piperazinyl)methyl)phenol, 3,5-Diamino-4-hydroxybrenzcatechin, 1,4-Bis-(4-aminophenyl)-1,4-diazacycloheptan, aromatische Nitrile, wie 2-Amino-4-hydroxy-benzonitril, 4-Amino-2-hydroxybenzonitril, 4-Aminobenzonitril, 2,4-Diaminobenzonitril, Nitrogruppen-haltige Aminoverbindungen, wie 3-Amino-6-methylamino-2-nitro-pyridin, Pikraminsäure, [8-[(4-Amino-2-nitrophenyl)-azo]-7-hydroxy-naphth-2-yl]-trimethylammoniumchlorid, [8-((4-Amino-3-nitrophenyl)-azo)-7-hydroxy-naphth-2-yl]-trimethylammoniumchlorid (Basic Brown 17), 1-Hydroxy-2-amino-4,6-dinitrobenzol, 1-Amino-2-nitro-4-[bis-(2-hydroxyethyl)amino]-benzol, 1-Amino-2-[(2-hydroxyethyl)amino]-5-nitrobenzol (HC Yellow Nr. 5), 1-Amino-2-nitro-4-[(2-hydroxyethyl)amino]-benzol (HC Red Nr.7), 2-Chlor-5-nitro-N-2-hydroxyethyl-1,4-phenylendiamin, 1-[(2-Hydroxyethyl)amino]-2-nitro-4-amino-benzol (HC Red Nr. 3), 4-Amino-3-nitrophenol, 4-Amino-2-nitrophenol, 6-Nitro-o-toluidin, 1-Amino-3-methyl-4-[(2-hydroxyethyl)amino]-6-nitrobenzol (HC Violet Nr. 1), 1-Amino-2-nitro-4-[(2,3-dihydroxypropyl)amino]-5-chlorbenzol (HC Red Nr.10), 2-(4-Amino-2-nitroanilino)-benzoesäure, 6-Nitro-2,5-diaminopyridin, 1-Amino-2-(3-nitrophenylazo)-7-phenylazo-8-naphthol-3,6-disulfonsäure Dinatriumsalz (Acid blue Nr. 29), 1-Amino-2-(2-hydroxy-4-nitrophenylazo)-8-naphthol-3,6-disulfonsäure Dinatriumsalz (Palatinchrome green), 1-Amino-2-(3-chlor-2-hydroxy-5-nitrophenylazo)-8-naphthol-3,6-disulfonsäure Dinatriumsalz (Gallion), 4-Amino-4'-nitrostilben-2,2'-disulfonsäure Dinatriumsalz, 2,4-Diamino-3',5'-dinitro-2'-hydroxy-5-methyl-azobenzol (Mordant brown 4), 4'-Amino-4-nitrodiphenylamin-2-sulfonsäure, 4'-Amino-3'-nitrobenzophenon-2-carbonsäure, 1-Amino-4-nitro-2-(2-nitrobenzylidenamino)-benzol, 2-[2-(Diethylamino)ethylamino]-5-nitroanilin, 3-Amino-4-hydroxy-5-nitrobenzolsulfonsäure, 3-Amino-3'-nitrobiphenyl, 3-Amino-4-nitro-acenaphthen, 2-Amino-1-nitronaphthalin, 5-Amino-6-nitrobenzo-1,3-dioxol, Aniline, insbesondere Nitrogruppen-haltige Aniline, wie 4-Nitroanilin, 2-Nitroanilin, 1,4-Diamino-2-nitrobenzol, 1,2-Diamino-4-nitrobenzol, 1-Amino-2-methyl-6-nitrobenzol, 4-Nitro-1,3-phenylendiamin, 2-Nitro-4-amino-1-(2-hydroxyethylamino)-benzol, 2-Nitro-1-amino-4-[bis-(2-hydroxyethyl)-amino]-benzol, 4-Amino-2-nitrodiphenylamin-2'-carbonsäure, 1-Amino-5-chlor-4-(2-hy-droyethylamino)-2-nitrobenzol, aromatische Aniline bzw. Phenole mit einem weiteren aromatischen Rest, wie sie in der Formel VII dargestellt sind 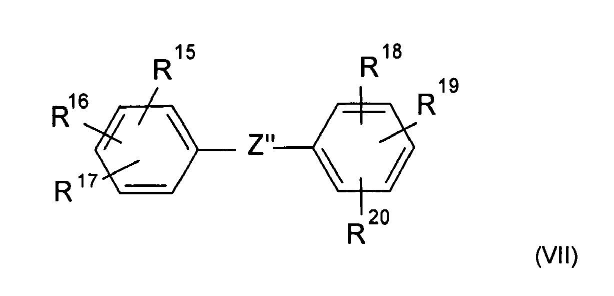 in der
- R¹⁵ für eine eine Aminogruppe, die durch eine C₁-C₆-Alkyl-, C₁-C₆-Hydroxyalkyl-, C₁-C₆-Alkoxy- oder C₁-C₆-Alkoxy-C₁-C₆-alkylgruppen substituiert sein kann, steht,
- R¹⁶, R¹⁷, R¹⁸, R¹⁹ und R²⁰ unabhängig voneinander für ein Wasserstoffatom, eine Hydroxy- oder eine Aminogruppe, die durch C₁-C₆-Alkyl-, C₁-C₆-Hydroxyalkyl-, C₁-C₆-Alkoxy-, C₁-C₆-Aminoalkyl- oder C₁-C₆-Alkoxy-C₁-C₆-alkylgruppen substituiert sein kann, stehen, und
- Z" für eine direkte Bindung, eine gesättigte oder ungesättigte, ggf. durch Hydroxygruppen substituierte Kohlenstoffkette mit 1 bis 4 Kohlenstoffatomen, eine Carbonyl-, Sulfonyl- oder Iminogruppe, ein Sauerstoff- oder Schwefelatom, oder eine Gruppe mit der Formel VIII 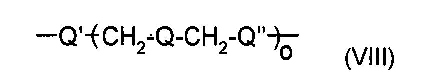 in der
- Q eine direkte Bindung, eine CH₂- oder CHOH-Gruppe bedeutet,
- Q' und Q" unabhängig voneinander für ein Sauerstoffatom, eine NR²¹-Gruppe, worin R²¹ ein Wasserstoffatom, eine C₁-C₆-Alkylgruppe oder C₁-C₆-Hydroxyalkylgruppe, wobei auch beide Gruppen zusammen mit dem Restmolekül einen 5-, 6- oder 7-Ring bilden können, bedeutet, die Gruppe O-(CH₂)ₚ-NH oder NH-(CH₂)ₚ'-O, worin p und p' 2 oder 3 sind, stehen und
- o eine Zahl von 1 bis 4 bedeutet,

wie insbesondere 4,4'-Diaminostilben und dessen Hydrochlorid, 4,4'-Diaminostilben-2,2'-disulfonsäure-mono- oder -di-Na-Salz, 4-Amino-4'-dimethylaminostilben und dessen Hydrochlorid, 4,4'-Diaminodiphenylmethan, 4,4'-Diaminodiphenylsulfid, 4,4'-Diaminodiphenylsulfoxid, 4,4'-Diaminodiphenylamin, 4,4'-Diaminodiphenylamin-2-sulfonsäure, 4,4'-Diaminobenzophenon, 4,4'-Diaminodiphenylether, 3,3',4,4'-Tetraaminodiphenyl, 3,3',4,4'-Tetraamino-benzophenon, 1,3-Bis-(2,4-diaminophenoxy)-propan, 1,8-Bis-(2,5-diaminophenoxy)-3,6-dioxaoctan, 1,3-Bis-(4-aminophenylamino)propan, , 1,3-Bis-(4-aminophenylamino)-2-propanol, 1,3-Bis-[N-(4-aminophenyl)-2-hydroxyethylamino]-2-propanol, N,N-Bis-[2-(4-aminophenoxy)-ethyl]-methylamin, N-Phenyl-1,4-phenylendiamin und Bis-(5-amino-2-hydroxyphenyl)-methan.

Als weitere primäre oder sekundäre, aromatische Amine sind entsprechende stickstoffhaltige Heteroaromaten mit primärer bzw. sekundärer Aminogruppe zu nennen. Diese sind bevorzugt ausgewählt aus der Gruppe bestehend aus 2-Aminopyridin, 3-Aminopyridin, 4-Aminopyridin, 2-Amino-3-hydroxy-pyridin, 2,6-Diamino-pyridin, 2,5-Diamino-pyridin, 2-(Aminoethylamino)-5-aminopyridin, 2,3-Diamino-pyridin, 2-Dimethylamino-5-amino-pyridin, 2-Methylamino-3-amino-6-methoxy-pyridin, 2,3-Diamino-6-methoxy-pyridin, 2,6-Dimethoxy-3,5-diamino-pyridin, 2,4,5-Triamino-pyridin, 2,6-Dihydroxy-3,4-dimethylpyridin, N-[2-(2,4-Diaminophenyl)aminoethyl]-N-(5-amino-2-pyridyl)-amin, N-[2-(4-Aminophenyl)aminoethyl]-N-(5-amino-2-pyridyl)-amin, 3,5-Diaminopyrazol, 3,5-Diamino-1,2,4-triazol, 3-Aminopyrazol, 3-Amino-5-hydroxypyrazol, 1-Phenyl-4,5-diaminopyrazol, 1-(2-Hydroxyethyl)-4,5-diaminopyrazol, 1-Phenyl-3-methyl-4,5-diaminopyrazol, 4-Amino-2,3-dimethyl-1-phenyl-3-pyrazolin-5-on (4-Aminoantipyrin), 1-Phenyl-3-methylpyrazol-5-on, 2-Aminochinolin, 3-Aminochinolin, 8-Aminochinolin, 2-Aminonicotinsäure, 6-Aminonicotinsäure, 5-Aminoisochinolin, 5-Aminoindazol, 6-Aminoindazol, 5-Aminobenzimidazol, 7-Aminobenzimidazol, 5-Aminobenzothiazol, 7-Aminobenzothiazol, 2,5-Dihydroxy-4-morpholino-anilin sowie Indolderivate, wie 4-Aminoindol, 5-Aminoindol, 6-Aminoindol, 7-Aminoindol und 5,6-Dihydroxyindol. Die vorgenannten Verbindungen können sowohl in freier Form als auch in Form ihrer physiologisch verträglichen Salze, z. B. als Salze anorganischer Säuren, wie Salz- oder Schwefelsäure, eingesetzt werden.

Erfindungsgemäß besonders bevorzugt werden die primären bzw. sekundären aromatischen Amine der Komponente (C) ausgewählt aus der Gruppe, die gebildet wird aus 2,5-Diamino-toluol, m-Phenylendiamin, o-Phenylendiamin, 2-(2-Hydroxyethyl)-p-phenylendiamin, N,N-Bis-(2-hydroxyethyl)-p-phenylendiamin, 3,5-Diamino-2-methoxy-1-methylbenzol, 2-(2,4-Diaminophenoxy)ethanol, 2-Amino-4-(2-hydroxyethyl)aminoanisol, 4-Aminophenol, 5-Amino-2-methylphenol, 2-Aminomethyl-4-aminophenol, 2-(Diethylamino)methyl-4-aminophenol, 5-Aminosalicylsäure, 3-Amino-2,4-dichlorphanol, 4-Amino-2,6-dichlorphenol, 5-Amino-4-chlor-2-methylphenol, 2-Amino-6-chlor-4-nitrophenol, 3-Amino-2-chlor-6-methylphenol, 2-Aminophenol, 1-Hydroxy-2-amino-5-methyl-benzol, 1-Hydroxy-2-amino-6-methyl-benzol, 3-Aminophenol, 4-Amino-3-methylphenol, 2-Amino-3-hydroxypyridin, 3-Amino-2-methylamino-6-methoxypyridin, 3,5-Diamino-2,6-dimethoxypyridin, 4,5-Diamino-1-(2-hydroxyethyl)-pyrazol, 1,3-Bis(2,4-diaminophenoxy)propan, Bis(5-amino-2-hydroxyphenyl)methan, 5,6-Dihydroxyindolin, 5,6-Dihydroxyindol, sowie den physiologisch verträglichen Salzen der genannten Verbindungen.

Die Komponente C kann jeweils in einer Menge von jeweils 0,03 bis 65 mmol, insbesondere 1 bis 40 mmol, jeweils bezogen auf 100 g der gesamten Anwendungsmischung, eingesetzt werden.

Zur Erlangung weiterer und intensiverer Ausfärbungen können die erfindungsgemäßen Mittel können zusätzlich Farbverstärker enthalten. Die Farbverstärker sind vorzugsweise ausgewählt aus der Gruppe bestehend aus Piperidin, Piperidin-2-carbonsäure, Piperidin-3-carbonsäure, Piperidin-4-carbonsäure, Pyridin, 2-Hydroxypyridin, 3-Hydroxypyridin, 4-Hydroxypyridin, Imidazol, 1-Methylimidazol, Histidin, Pyrrolidin, Prolin, Pyrrolidon, Pyrrolidon-5-carbonsäure, Pyrazol, 1,2,4-Triazol, Piperazidin, deren Derivate sowie deren physiologisch verträglichen Salzen.

Die voranstehend genannten Farbverstärker können in einer Menge von jeweils 0,03 bis 65 mmol, insbesondere 1 bis 40 mmol, jeweils bezogen auf 100 g des gesamten Färbemittels, eingesetzt werden.

Für das Färbeergebnis kann es vorteilhaft sein, den Färbemitteln Ammonium- oder Metallsalze zuzugeben. Geeignete Metallsalze sind z.B. Formiate, Carbonate, Halogenide, Sulfate, Butyrate, Valeriate, Capronate, Acetate, Lactate, Glykolate, Tartrate, Citrate, Gluconate, Propionate, Phosphate und Phosphonate von Erdalkalimetallen, wie Kalium, Natrium oder Lithium, Erdalkalimetallen, wie Magnesium, Calcium, Strontium oder Barium, oder von Aluminium, Mangan, Eisen, Kobalt, Kupfer oder Zink, wobei Natriumacetat, Lithiumbromid, Calcium bromid, Calciumgluconat, Zinkchlorid, Zinksulfat, Magnesiumchlorid, Magnesiumsulfat, Ammoniumcarbonat, -chlorid und -acetat bevorzugt sind. Diese Salze sind vorzugsweise in einer Menge von 0,03 bis 65, insbesondere von 1 bis 40 mmol, bezogen auf 100 g des gesamten Färbemittels, enthalten.

Auf die Anwesenheit von zusätzlichen chemischen Oxidationsmitteln, z.B. H₂O₂, wird bevorzugt in den erfindungsgemäßen Färbemitteln verzichtet. Es kann jedoch u.U. wünschenswert sein, den erfindungsgemäßen Mitteln zur Erzielung von Nuancen, die heller als die zu färbende keratinhaltige Faser sind, Wasserstoffperoxid oder andere Oxidationsmittel zuzusetzen. Oxidationsmittel werden in der Regel in einer Menge von 0,01 bis 6 Gew.-%, bezogen auf die Anwendungslösung, eingesetzt. Ein für menschliches Haar bevorzugtes Oxidationsmittel ist H₂O₂.

Desweiteren können die erfindungsgemäßen Färbemittel zur Nuancierung einen oder mehrere direktziehende Farbstoffe enthalten. Direktziehende Farbstoffe sind üblicherweise Nitrophenylendiamine, Nitroaminophenole, Azofarbstoffe, Anthrachinone oder Indophenole. Bevorzugte direktziehende Farbstoffe sind die unter den internationalen Bezeichnungen bzw. Handelsnamen HC Yellow 2, HC Yellow 4, HC Yellow 5, HC Yellow 6, HC Yellow 12, Acid Yellow 1, Acid Yellow 10, Acid Yellow 23, Acid Yellow 36, HC Orange 1, Disperse Orange 3, Acid Orange 7, HC Red 1, HC Red 3, HC Red 10, HC Red 11, HC Red 13, Acid Red 33, Acid Red 52, HC Red BN, Pigment Red 57:1, HC Blue 2, HC Blue 12, Disperse Blue 3, Acid Blue 7, Acid Green 50, HC Violet 1, Disperse Violet 1, Disperse Violet 4, Acid Violet 43, Disperse Black 9, Acid Black 1, und Acid Black 52 bekannten Verbindungen sowie 1,4-Diamino-2-nitrobenzol, 2-Amino-4-nitrophenol, 1,4-Bis-(β-hydroxyethyl)amino-2-nitrobenzol, 3-Nitro-4-(β-hydroxyethyl)aminophenol, 2-(2'-Hydroxyethyl)amino-4,6-dinitrophenol, 1-(2'-Hydroxyethyl)amino-4-methyl-2-nitrobenzol, 1-Amino-4-(2'-hydroxyethyl)amino-5-chlor-2-nitrobenzol, 4-Amino-3-nitrophenol, 1-(2'-Ureidoethyl)amino-4-nitrobenzol, 4-Amino-2-nitrodiphenylamin-2'-carbonsäure, 6-Nitro-1,2,3,4-tetrahydrochinoxalin, 2-Hydroxy-1,4-naphthochinon, Pikraminsäure und deren Salze, 4-Ethylamino-3-nitrobenzoesäure und 2-Chloro-6-ethylamino-1-hydroxy-4-nitrobenzol.

Ferner können die erfindungsgemäßen Mittel einen kationischen direktziehenden Farbstoff enthalten. Besonders bevorzugt sind dabei
(a) kationische Triphenylmethanfarbstoffe, wie beispielsweise Basic Blue 7, Basic Blue 26, Basic Violet 2 und Basic Violet 14,
(b) aromatischen Systeme, die mit einer quaternären Stickstoffgruppe substituiert sind, wie beispielsweise Basic Yellow 57, Basic Red 76, Basic Blue 99, Basic Brown 16 und Basic Brown 17, sowie
(c) direktziehende Farbstoffe, die einen Heterozyklus enthalten, der mindestens ein quaternäres Stickstoffatom aufweist, wie sie beispielsweise in der EP-A2-998 908, auf die an dieser Stelle explizit Bezug genommen wird, in den Ansprüchen 6 bis 11 genannt werden.

Bevorzugte kationische direktziehende Farbstoffe der Gruppe (c) sind insbesondere die folgenden Verbindungen:

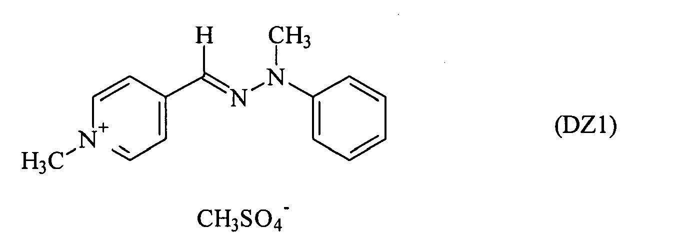

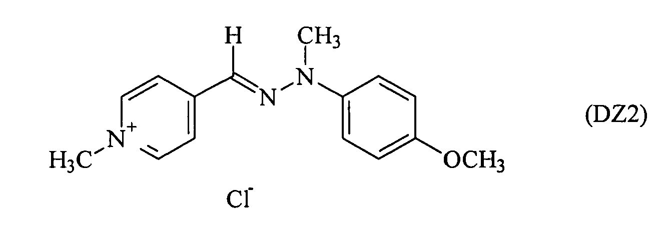

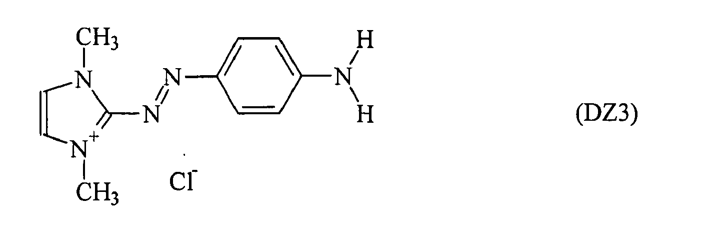

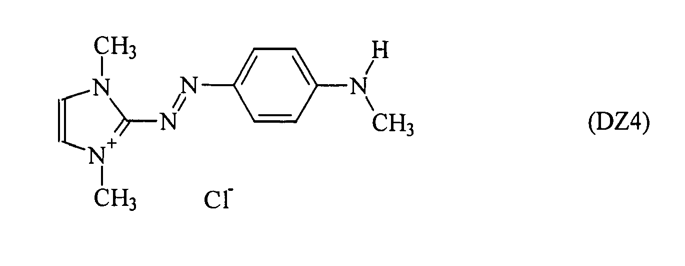

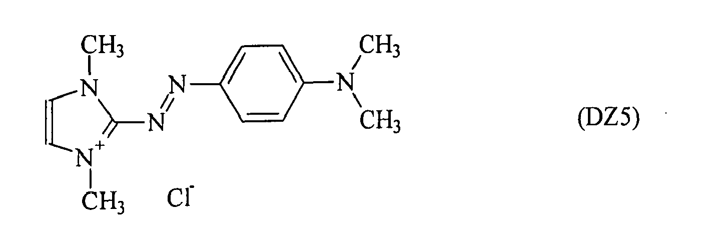

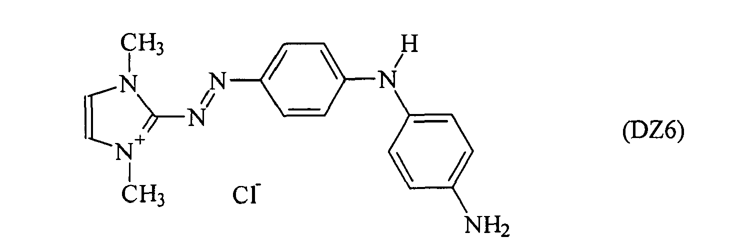

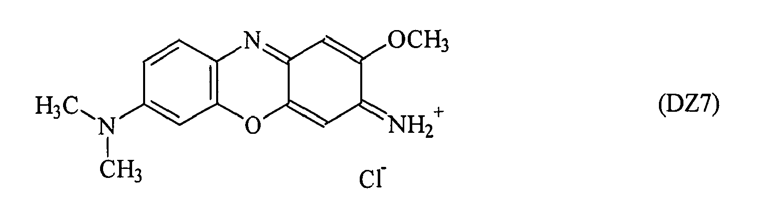

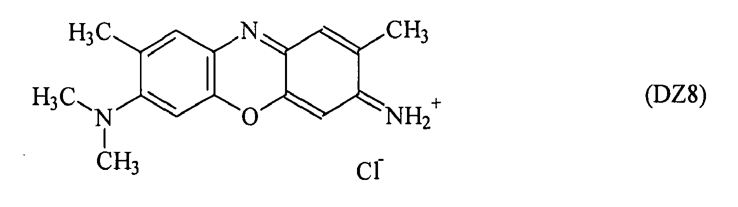

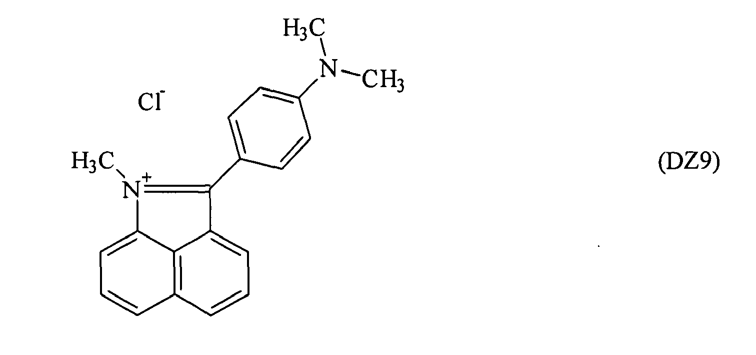

Die Verbindungen der Formeln (DZ1), (DZ3) und (DZ5), die auch unter den Bezeichnungen Basic Yellow 87, Basic Orange 31 und Basic Red 51 bekannt sind, sind ganz besonders bevorzugte kationische direktziehende Farbstoffe der Gruppe (c).

Die kationischen direktziehenden Farbstoffe, die unter dem Warenzeichen Arianor^{®} vertrieben werden, sind erfindungsgemäß ebenfalls ganz besonders bevorzugte kationische direktziehende Farbstoffe.

Die erfindungsgemäßen Mittel gemäß dieser Ausführungsform enthalten die direktziehenden Farbstoffe bevorzugt in einer Menge von 0,01 bis 20 Gew.-%, bezogen auf das gesamte Färbemittel.

Weiterhin können die erfindungsgemäßen Färbemittel auch in der Natur vorkommende Farbstoffe wie sie beispielsweise in Henna rot, Henna neutral, Henna schwarz, Kamillenblüte, Sandelholz, schwarzem Tee, Faulbaumrinde, Salbei, Blauholz, Krappwurzel, Catechu, Sedre und Alkannawurzel enthalten sind, enthalten.

Die erfindungsgemäßen Färbemittel ergeben bereits bei physiologisch verträglichen Temperaturen von unter 45°C intensive Färbungen. Sie eignen sich deshalb besonders zum Färben von menschlichen Haaren. Zur Anwendung auf dem menschlichen Haar können die erfindungsgemäße Zusammensetzungenüblicherweise in einen wasserhaltigen kosmetischen Träger eingearbeitet werden. Geeignete wasserhaltige kosmetische Träger sind z.B. Cremes, Emulsionen, Gele oder auch tensidhaltige schäumende Lösungen wie z.B. Shampoos oder andere Zubereitungen, die für die Anwendung auf den keratinhaltigen Fasern geeignet sind.

Die erfindungsgemäßen Mittel enthalten in einer weiteren Ausführungsform zusätzlich mindestens ein lineares oder verzweigtes, aliphatisches Polyol, bevorzugt in einer Menge von 2 bis 50 Gew.-%, besonders bevorzugt in einer Menge von 5 bis 30 Gew.-%, jeweils bezogen auf das Gewicht des gesamten Färbemittels.

Bevorzugte Vertreter der erfindungsgemäß verwendbaren Polyole werden aus einer Gruppe ausgewählt, die gebildet wird aus HO-(CH₂CH₂O)ₙ-H mit n = 1 bis 14, Dipropylenglykol, Tripropylenglykol, Glycerin, Diglycerin, 1,2-Butandiol, 1,4-Butandiol, 1,5-Pentandiol, 1,2-Pentandiol, 2-Methyl-2,4-pentandiol, 4-Methyl-2-pentanol, 1,2,6-Hexantriol und 2-Ethyl-1,3-hexandiol. Ein besonders bevorzugtes Polyol ist Ethylenglykol.

Zusätzlich können die erfindungsgemäßen Färbemittel mindestens ein Polymer, ausgewählt aus der Gruppe, die gebildet wird aus anionischen, amphoteren, kationischen und nichtionischen Polymeren, insbesondere aus kationischen und nichtionischen Polymeren. Dabei kann es wiederum bevorzugt sein, wenn die oben genannten Polymertypen von Polysacchariden und dessen Derivaten abgeleitet sind.

Bei den verwendbaren anionischen Polymeren, handelt es sich um ein anionische Polymere, welche Carboxylat- und/oder Sulfonatgruppen und/oder Phosphatgruppen aufweisen. Beispiele für anionische Monomere, aus denen derartige Polymere bestehen können, sind Acrylsäure, Methacrylsäure, Crotonsäure, Maleinsäureanhydrid und 2-Acrylamido-2-methylpropansulfonsäure. Dabei können die sauren Gruppen ganz oder teilweise als Natrium-, Kalium-, Ammonium-, Mono- oder Triethanolammonium-Salz vorliegen. Bevorzugte Monomere sind 2-Acrylamido-2-methylpropansulfonsäure und Acrylsäure.

Ein Beispiel für ein anionisches Polymer mit einer Phosphatgruppe ist das unter dem Handelsnamen Structure^{®} Zea vertriebene Polysaccharidderivat (NATIONAL STARCH) (INCI-Bezeichnung: Hydroxypropyl Starch Phosphate).

Weitere verwendbare anionische Polymere, enthalten als alleiniges oder Co-Monomer 2-Acrylamido-2-methylpropansulfonsäure, wobei die Sulfonsäuregruppe ganz oder teilweise als Natrium-, Kalium-, Ammonium-, Mono- oder Triethanolammonium-Salz vorliegen kann.

Besonders bevorzugt ist das Homopolymer der 2-Acrylamido-2-methylpropansulfonsäure, das beispielsweise unter der Bezeichnung Rheothik^{®}11-80 im Handel erhältlich ist.

Innerhalb dieser Ausführungsform kann es bevorzugt sein, Copolymere aus mindestens einem anionischen Monomer und mindestens einem nichtionogenen Monomer einzusetzen. Bezüglich der anionischen Monomere wird auf die oben aufgeführten Substanzen verwiesen. Bevorzugte nichtionogene Monomere sind Acrylamid, Methacrylamid, Acrylsäureester, Methacrylsäureester, Vinylpyrrolidon, Vinylether und Vinylester.

Weitere anionische Copolymere sind Acrylsäure-Acrylamid-Copolymere sowie insbesondere Polyacrylamidcopolymere mit Sulfonsäuregruppen-haltigen Monomeren. Ein solches anionisches Copolymer besteht bevorzugt aus 70 bis 55 Mol-% Acrylamid und 30 bis 45 Mol-% 2-Acrylamido-2-methylpropansulfonsäure, wobei die Sulfonsäuregruppe ganz oder teilweise als Natrium-, Kalium-, Ammonium-, Mono- oder Triethanolammonium-Salz vorliegt. Dieses Copolymer kann auch vernetzt vorliegen, wobei als Vernetzungsagentien bevorzugt polyolefinisch ungesättigte Verbindungen wie Tetraallyloxyethan, Allylsucrose, Allylpentaerythrit und Methylen-bisacrylamid zum Einsatz kommen. Ein solches Polymer ist in dem Handelsprodukt Sepigel^{®}305 der Firma SEPPIC enthalten. Die Verwendung dieses Compounds, das neben der Polymerkomponente eine Kohlenwasserstoffmischung (C₁₃-C₁₄-Isoparaffin) und einen nichtionogenen Emulgator (Laureth-7) enthält, hat sich im Rahmen der erfindungsgemäßen Lehre als besonders vorteilhaft erwiesen.

Auch die unter der Bezeichnung Simulgel^{®}600 als Compound mit Isohexadecan und Polysorbat-80 vertriebenen Natriumacryloyldimethyltaurat-Copolymere haben sich als erfindungsgemäß besonders wirksam erwiesen.

Ebenfalls bevorzugte anionische Homopolymere sind unvernetzte und vernetzte Polyacrylsäuren. Dabei können Allylether von Pentaerythrit, von Sucrose und von Propylen bevorzugte Vernetzungsagentien sein. Solche Verbindungen sind beispielsweise unter dem Warenzeichen Carbopol^{®} im Handel erhältlich.

Copolymere aus Maleinsäureanhydrid und Methylvinylether, insbesondere solche mit Vernetzungen, sind ebenfalls farberhaltende Polymere. Ein mit 1,9-Decadiene vernetztes Maleinsäure-Methylvinylether-Copolymer ist unter der Bezeichnung Stabileze^{®} QM im Handel erhältlich.

Weiterhin können als Polymere amphotere Polymere eingesetzt werden. Unter dem Begriff amphotere Polymere werden sowohl solche Polymere, die im Molekül sowohl freie Aminogruppen als auch freie -COOH- oder SO₃H-Gruppen enthalten und zur Ausbildung innerer Salze befähigt sind, als auch zwitterionische Polymere, die im Molekül quartäre Ammoniumgruppen und -COO- oder -SO₃-Gruppen enthalten, und solche Polymere zusammengefaßt, die -COOH- oder SO₃H-Gruppen und quartäre Ammoniumgruppen enthalten.

Ein Beispiel für ein erfindungsgemäß einsetzbares Amphopolymer ist das unter der Bezeichnung Amphomer^{®} erhältliche Acrylharz, das ein Copolymeres aus tert.-Butylaminoethylmethacrylat, N-(1,1,3,3-Tetramethylbutyl)acrylamid sowie zwei oder mehr Monomeren aus der Gruppe Acrylsäure, Methacrylsäure und deren einfachen Estern darstellt.

Weitere erfindungsgemäß einsetzbare amphotere Polymere sind die in der britischen Offenlegungsschrift 2 104 091, der europäischen Offenlegungsschrift 47 714, der europäischen Offenlegungsschrift 217 274, der europäischen Offenlegungsschrift 283 817 und der deutschen Offenlegungsschrift 28 17 369 genannten Verbindungen.

Bevorzugt eingesetzte amphotere Polymere sind solche Polymerisate, die sich im wesentlichen zusammensetzen aus
(a) Monomeren mit quartären Ammoniumgruppen der allgemeinen Formel P1,

   R¹-CH=CR²-CO-Z-(CₙH₂ₙ)-N⁽⁺⁾R³R⁴R⁵ A⁽⁻⁾ (P1)

   in der R¹ und R² unabhängig voneinander stehen für Wasserstoff oder eine Methylgruppe und R³, R⁴ und R⁵ unabhängig voneinander für Alkylgruppen mit 1 bis 4 Kohlenstoff-Atomen, Z eine NH-Gruppe oder ein Sauerstoffatom, n eine ganze Zahl von 2 bis 5 und A⁽⁻⁾ das Anion einer organischen oder anorganischen Säure ist
   und
(b) monomeren Carbonsäuren der allgemeinen Formel P2,

   R¹-CH=CR²-COOH (P2)

   in denen R¹ und R² unabhängig voneinander Wasserstoff oder Methylgruppen sind.

Diese Verbindungen können sowohl direkt als auch in Salzform, die durch Neutralisation der Polymerisate, beispielsweise mit einem Alkalihydroxid, erhalten wird, erfindungsgemäß eingesetzt werden. Bezüglich der Einzelheiten der Herstellung dieser Polymerisate wird ausdrücklich auf den Inhalt der deutschen Offenlegungsschrift 39 29 973 Bezug genommen. Ganz besonders bevorzugt sind solche Polymerisate, bei denen Monomere der Formel P1 eingesetzt werden, bei denen R³, R⁴ und R⁵ Methylgruppen sind, Z eine NH-Gruppe und A⁽⁻⁾ ein Halogenid-, Methoxysulfat- oder Ethoxysulfat-Ion ist; Acrylamidopropyl-trimethyl-ammoniumchlorid ist ein besonders bevorzugtes Monomeres gemäß Formel P1. Als Monomeres der Formel P2 für die genannten Polymerisate wird bevorzugt Acrylsäure verwendet.

Unter kationischen Polymeren sind Polymere zu verstehen, welche in der Haupt-und/oder Seitenkette Gruppen aufweisen, welche "temporär" oder "permanent" kationisch sein kann. Als "permanent kationisch" werden erfindungsgemäß solche Polymere bezeichnet, die unabhängig vom pH-Wert der Mittel eine kationische Gruppe aufweisen. Dies sind in der Regel Polymere, die ein quartäres Stickstoffatom, beispielsweise in Form einer Ammoniumgruppe, enthalten. Bevorzugte kationische Gruppen sind quartäre Ammoniumgruppen. Insbesondere solche Polymere, bei denen die quartäre Ammoniumgruppe über eine C₁₋₄-Kohlenwasserstoffgruppe an eine aus Acrylsäure, Methacrylsäure oder deren Derivaten aufgebaute Polymerhauptkette gebunden sind, haben sich als besonders geeignet erwiesen.

### Homopolymere der allgemeinen Formel P3,

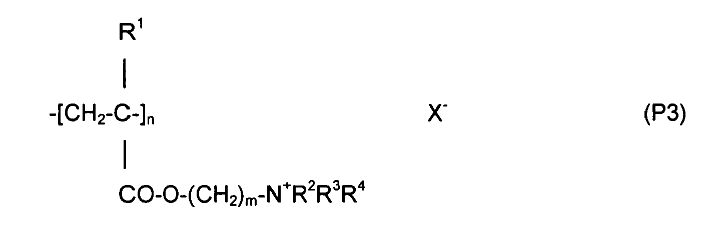

in der R¹ = -H oder -CH₃ ist, R², R³ und R⁴ unabhängig voneinander ausgewählt sind aus C₁₋₄-Alkyl-, -Alkenyl- oder -Hydroxyalkylgruppen, m = 1, 2, 3 oder 4, n eine natürliche Zahl und X⁻ ein physiologisch verträgliches organisches oder anorganisches Anion ist, sowie Copolymere, bestehend im wesentlichen aus den in Formel P3 aufgeführten Monomereinheiten sowie nichtionogenen Monomereinheiten, sind besonders bevorzugte kationische Polymere. Im Rahmen dieser Polymere sind diejenigen erfindungsgemäß bevorzugt, für die mindestens eine der folgenden Bedingungen gemäß Formel P3 gilt:
- R¹ steht für eine Methylgruppe
- R², R³ und R⁴ stehen für Methylgruppen
- m hat den Wert 2.

Als physiologisch verträgliches Gegenion X⁻ gemäß Formel P3 kommen beispielsweise Halogenidionen, Sulfationen, Phosphationen, Methosulfationen sowie organische Ionen wie Lactat-, Citrat-, Tartrat- und Acetationen in Betracht. Bevorzugt sind Halogenidionen, insbesondere Chlorid.

Ein besonders geeignetes Homopolymer ist das, gewünschtenfalls vernetzte, Poly(methacryloyloxyethyltrimethylammoniumchlorid) mit der INCI-Bezeichnung Polyquaternium-37. Die Vernetzung kann gewünschtenfalls mit Hilfe mehrfach olefinisch ungesättigter Verbindungen, beispielsweise Divinylbenzol, Tetraallyloxyethan, Methylen-bisacrylamid, Diallylether, Polyallylpolyglycerylether, oder Allylethern von Zuckern oder Zuckerderivaten wie Erythritol, Pentaerythritol, Arabitol, Mannitol, Sorbitol, Sucrose oder Glucose erfolgen. Methylenbisacrylamid ist ein bevorzugtes Vernetzungsagens.

Das Homopolymer wird bevorzugt in Form einer nichtwäßrigen Polymerdispersion, die einen Polymeranteil nicht unter 30 Gew.-% aufweisen sollte, eingesetzt. Solche Polymerdispersionen sind unter den Bezeichnungen Salcare^{®} SC 95 (ca. 50 % Polymeranteil, weitere Komponenten: Mineralöl (INCI-Bezeichnung: Mineral Oil) und Tridecyl-polyoxypropylen-polyoxyethylen-ether (INCI-Bezeichnung: PPG-1-Trideceth-6)) und Salcare^{®} SC 96 (ca. 50 % Polymeranteil, weitere Komponenten: Mischung von Diestern des Propylenglykols mit einer Mischung aus Capryl- und Caprinsäure (INCI-Bezeichnung: Propylene Glycol Dicaprylate/Dicaprate) und Tridecyl-polyoxypropylen-polyoxyethylen-ether (INCI-Bezeichnung: PPG-1-Trideceth-6)) im Handel erhältlich.

Copolymere mit Monomereinheiten gemäß Formel P3 enthalten als nichtionogene Monomereinheiten bevorzugt Acrylamid, Methacrylamid, Acrylsäure-C₁₋₄-alkylester und Methacrylsäure-C₁₋₄-alkylester. Unter diesen nichtionogenen Monomeren ist das Acrylamid besonders bevorzugt. Auch diese Copolymere können, wie im Falle der Homopolymere oben beschrieben, vernetzt sein. Ein erfindungsgemäß bevorzugtes Copolymer ist das vernetzte Acrylamid-Methacryloyloxyethyltrimethylammoniumchlorid-Copolymer. Solche Copolymere, bei denen die Monomere in einem Gewichtsverhältnis von etwa 20:80 vorliegen, sind im Handel als ca. 50 %ige nichtwäßrige Polymerdispersion unter der Bezeichnung Salcare^{®} SC 92 erhältlich.

Weitere bevorzugte kationische Polymere sind beispielsweise
- quaternisierte Cellulose-Derivate, wie sie unter den Bezeichnungen Celquat^{®} und Polymer JR^{®} im Handel erhältlich sind. Die Verbindungen Celquat^{®} H 100, Celquat^{®} L 200 und Polymer JR^{®}400 sind bevorzugte quaternierte Cellulose-Derivate,
- kationische Alkylpolyglycoside gemäß der DE-PS 44 13 686,
- kationiserter Honig, beispielsweise das Handelsprodukt Honeyquat^{®} 50,
- kationische Guar-Derivate, wie insbesondere die unter den Handelsnamen Cosmedia^{®}Guar (COGNIS), N-Hance^{®} 3000, N-Hance^{®} 3196, N-Hance^{®} 3205, N-Hance^{®} 3215 (alle AQUALON) und Jaguar^{®} (RHODIA) vertriebenen Produkte,
- Polysiloxane mit quaternären Gruppen, wie beispielsweise die im Handel erhältlichen Produkte Q2-7224 (Hersteller: Dow Corning; ein stabilisiertes Trimethylsilylamodimethicon), Dow Corning^{®} 929 Emulsion (enthaltend ein hydroxyl-aminomodifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80),
- polymere Dimethyldiallylammoniumsalze und deren Copolymere mit Estern und Amiden von Acrylsäure und Methacrylsäure. Die unter den Bezeichnungen Merquat^{®}100 (Poly(dimethyldiallylammoniumchlorid)) und Merquat^{®}550 (Dimethyldiallylammoniumchlorid-Acrylamid-Copolymer) im Handel erhältlichen Produkte sind Beispiele für solche kationischen Polymere,
- Copolymere des Vinylpyrrolidons mit quaternierten Derivaten des Dialkylaminoalkylacrylats und -methacrylats, wie beispielsweise mit Diethylsulfat quaternierte Vinylpyrrolidon-Dimethylaminoethylmethacrylat-Copolymere. Solche Verbindungen sind unter den Bezeichnungen Gafquat^{®}734 und Gafquat^{®}755 im Handel erhältlich,
- Vinylpyrrolidon-Vinylimidazoliummethochlorid-Copolymere, wie sie unter den Bezeichnungen Luviquat^{®} FC 370, FC 550, FC 905 und HM 552 angeboten werden.
- quaternierter Polyvinylalkohol,
   sowie die unter den Bezeichnungen
- Polyquaternium 2,
- Polyquaternium 17,
- Polyquaternium 18 und
- Polyquaternium 27 bekannten Polymeren mit quartären Stickstoffatomen in der Polymerhauptkette.

Gleichfalls als kationische Polymere eingesetzt werden können die unter den Bezeichnungen Polyquaternium-24 (Handelsprodukt z. B. Quatrisoft^{®} LM 200), bekannten Polymere. Ebenfalls erfindungsgemäß verwendbar sind die Copolymere des Vinylpyrrolidons, wie sie als Handelsprodukte Copolymer 845 (Hersteller: ISP), Gaffix^{®} VC 713 (Hersteller: ISP), Gafquat^{®}ASCP 1011, Gafquat^{®}HS 110, Luviquat^{®}8155 und Luviquat^{®} MS 370 erhältlich sind.

Weitere erfindungsgemäße kationische Polymere sind die sogenannten "temporär kationischen" Polymere. Diese Polymere enthalten üblicherweise eine Aminogruppe, die bei bestimmten pH-Werten als quartäre Ammoniumgruppe und somit kationisch vorliegt. Bevorzugt sind beispielsweise Chitosan und dessen Derivate, wie sie beispielsweise unter den Handelsbezeichnungen Hydagen^{®} CMF, Hydagen^{®} HCMF, Kytamer^{®} PC und Chitolam^{®} NB/101 im Handel frei verfügbar sind. Chitosane sind deacetylierte Chitine, die in unterschiedlichen Deacetylierungsgraden und unterschiedlichen Abbaugraden (Molekulargewichten) im Handel erhältlich sind. Ihre Herstellung ist z.B. in DE 44 40 625 A1 und in DE 1 95 03 465 A1 beschrieben.

Besonders gut geeignete Chitosane weisen einen Deacetylierungsgrad von wenigstens 80 % und ein Molekulargewicht von 5·10⁵ bis 5·10⁶ (g/mol) auf.

Zur Herstellung erfindungsgemäßer Zubereitungen muß das Chitosan in die Salzform überführt werden. Dies kann durch Auflösen in verdünnten wäßrigen Säuren erfolgen. Als Säuren sind sowohl Mineralsäuren wie z.B. Salzsäure, Schwefelsäure und Phosphorsäure als auch organische Säuren, z.B. niedermolekulare Carbonsäuren, Polycarbonsäuren und Hydroxycarbonsäuren geeignet. Weiterhin können auch höhermolekulare Alkylsulfonsäuren oder Alkylschwefelsäuren oder Organophosphorsäuren verwendet werden, soweit diese die erforderliche physiologische Verträglichkeit aufweisen. Geeignete Säuren zur Überführung des Chitosans in die Salzform sind z.B. Essigsäure, Glycolsäure, Weinsäure, Apfelsäure, Citronensäure, Milchsäure, 2-Pyrrolidinon-5-carbonsäure, Benzoesäure oder Salicylsäure. Bevorzugt werden niedermolekulare Hydroxycarbonsäuren wie z.B. Glycolsäure oder Milchsäure verwendet.

Bevorzugt verwendete nichtionogene Polymere werden ausgewählt aus:
- Vinylpyrrolidon/Vinylester-Copolymere, wie sie beispielsweise unter dem Warenzeichen Luviskol^{®} (BASF) vertrieben werden. Luviskol^{®} VA 64 und Luviskol^{®} VA 73, jeweils Vinylpyrrolidon/Vinylacetat-Copolymere, sind ebenfalls bevorzugte nichtionische Polymere.
- Cellulose und Derivate davon, insbesondere Celluloseether, wie Hydroxypropylcellulose, Hydroxyethylcellulose und Methylhydroxypropylcellulose, wie sie beispielsweise unter den Warenzeichen Klucel^{®} (INCI-Bezeichnung: Hydroxypropylcellulose), Culminal^{®} (INCI-Bezeichnung: Hydroxypropylcellulose) und Benecel^{®} (INCI-Bezeichnung: Hydroxypropyl Methylcellulose) (alle AQUALON) sowie Natrosol^{®} (AQUALON), insbesondere Natrosol^{®} 250 HR (INCI-Bezeichnung: Hydroxyethylcellulose) und Natrosol^{®} Plus 330 CS (INCI-Bezeichnung: Cetylhydroxyethylcellulose) vertrieben werden.
- Guar und Derivate davon, wie sie beispielsweise unter der Warenzeichen N-Hance^{®} HP-40 (AQUALON) vertrieben werden
- Stärke-Fraktionen und Derivate davon, wie beispielsweise Amylose, Amylopektin und Dextrine, die z.B. unter dem Handelsnamen Amaze^{®} und Structure^{®} Solanance (NATIONAL STARCH) erhältlich sind- Schellack
- Polyvinylpyrrolidone, wie sie beispielsweise unter der Bezeichnung Luviskol^{®} (BASF) vertrieben werden.
- Agar-Agar, Alginate, Xanthan-Gum, Gummi arabicum, Karaya-Gummi, Johannisbrotkernmehl, Leinsamengummen, Dextrane.

Es ist besonders bevorzugt, solche nichtionischen Polymere zu verwenden, welche sich von einem Polysaccharid ableiten, wie Cellulose und seine Derivate, Stärke-Fraktionen und deren Derivate, Guar und seine Derivate.

Die Polymere werden erfindungsgemäß bevorzugt in Mengen von 0,05 bis 5 Gew.-%, bezogen auf die gesamte Zusammensetzung, eingesetzt. Mengen von 0,25 bis 2 Gew.% sind besonders bevorzugt.

Weiterhin können die erfindungsgemäßen Mittel alle in solchen Zubereitungen bekannten Wirk-, Zusatz- und Hilfsstoffe enthalten. In vielen Fällen enthalten die Mittel mindestens ein Tensid, wobei prinzipiell sowohl anionische als auch zwitterionische, ampholytische, nichtionische und kationische Tenside geeignet sind. In vielen Fällen hat es sich aber als vorteilhaft erwiesen, die Tenside aus anionischen, zwitterionischen oder nichtionischen Tensiden auszuwählen.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ -CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-SO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül sowie insbesondere Salze von gesättigten und insbesondere ungesättigten C₈-C₂₂-Carbonsäuren, wie Ölsäure, Stearinsäure, Isostearinsäure und Palmitinsäure.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethyl-ammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer C₈₋₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Nichtionische Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂₋₂₂-Fettsäuremono- und -diester von Anlagerungsprodukten von 1 bis 30 Mol Ethylenoxid an Glycerin,
- C₈₋₂₂-Alkylmono- und -oligoglycoside und deren ethoxylierte Analoga,
- Anlagerungsprodukte von 5 bis 60 Mol Ethylenoxid an Rizinusöl und gehärtetes Rizinusöl,
- Anlagerungeprodukte von Ethylenoxid an Sorbitanfettsäureester
- Anlagerungsprodukte von Ethylenoxid an Fettsäurealkanolamide.

Beispiele für die in den erfindungsgemäßen Mitteln verwendbaren kationischen Tenside sind insbesondere quartäre Ammoniumverbindungen. Bevorzugt sind Ammoniumhalogenide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Erfindungsgemäß ebenfalls geeignet sind kationische Silikonöle wie beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxylamino-modifiziertes Silicon, das auch als Amodimethicone bezeichnet wird), SM-2059 (Hersteller: General Electric), SLM-55067 (Hersteller: Wacker) sowie Abil^{®}-Quat 3270 und 3272 (Hersteller: Th. Goldschmidt; diquaternäre Polydimethylsiloxane, Quaternium-80).

Alkylamidoamine, insbesondere Fettsäureamidoamine wie das unter der Bezeichnung Tego Amid^{®}S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind quaternäre Esterverbindungen, sogenannte "Esterquats", wie die unter dem Warenzeichen Stepantex^{®} vertriebenen Methylhydroxyalkyldialkoyloxyalkylammoniummethosulfate.

Ein Beispiel für ein als kationisches Tensid einsetzbares quaternäres Zuckerderivat stellt das Handelsprodukt Glucquat^{®}100 dar, gemäß CTFA-Nomenklatur ein "Lauryl Methyl Gluceth-10 Hydroxypropyl Dimonium Chloride".

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Die Tenside werden bevorzugt in Konzentrationen von 0,5 bis 30 Gew.-%, bezogen auf das Gewicht der Zusammensetzung, eingesetzt.

Die erfindungsgemäßen Mittel können zusätzlich einen Fettalkohol enthalten. Unter Fettalkoholen sind primäre aliphatische Monoalkohole mit einem aliphatischen, linearen oder verzweigten Kohlenwasserstoffrest mit 6 bis 30 Kohlenstoffatomen und 0 und/oder 1, 2 oder 3 Doppelbindungen steht. Typische Beispiele sind Ca-pronalkohol, Caprylalkohol, 2-Ethylhexylalkohol, Caprinalkohol, Laurylalkohol, Isotridecylalkohol, Myristylalkohol, Cetylalkohol, Palmoleylalkohol, Stearylalkohol, Isostearylalkohol, Oleylalkohol, Elaidylalko-hol, Petroselinylalkohol, Linolylalkohol, Linolenylalkohol, Elaeostearylalkohol, Arachylalkohol, Gadoleyl-alkohol, Behenylalkohol, Erucylalkohol und Brassidylalkohol sowie deren technische Mischungen, die z.B. bei der Hochdruckhydrierung von technischen Methylestern auf Basis von Fetten und Ölen oder Aldehyden aus der Roelen'schen Oxosynthese sowie als Monomerfraktion bei der Dimerisierung von ungesättigten Fettalkoholen anfallen. Bevorzugt sind technische Fettalkohole mit 12 bis 18 Kohlenstoffatomen, wie beispielsweise Kokos-, Palm-, Palmkern- oder Talgfettalkohol.

Die erfindungsgemäßen Mittel können zusätzlich einen Ölkörper enthalten. Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen C₆-C₂₂-Fettalkoholen, Ester von verzweigten C₆-C₁₃-Carbonsäuren mit linearen C₆-C₂₂-Fettalkoholen, Ester von linearen C₆-C₂₂-Fettsäuren mit verzweigten Alkoholen, insbesondere 2-Ethylhexanol, Ester von linearen und/oder verzweigten Fettsäuren mit mehrwertigen Alkoholen (wie z.B. Propylenglycol, Dimer-diol oder Trimertriol) und/oder Guerbetalkoholen, Triglyceride auf Basis C₆-C₁₀-Fettsäuren, flüssige Mono-/Di/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, pflanzliche Öle, verzweigte primäre Alkohole, substituierte Cyclohexane, lineare C₆-C₂₂-Fettalkoholcarbonate, Guerbetcarbonate, Ester der Benzoesäure mit linearen und/oder verzweigten C₆-C₂₂-Alkoholen (z.B. Finsolv^{®} TN), Dialkylether, Ringöffnungsprodukte von epoxidierten Fettsäureestern mit Polyolen, Siliconöle und/oder aliphatische bzw. naphthenische Kohlenwasserstoffe in Betracht.

Weitere Wirk-, Hilfs- und Zusatzstoffe sind beispielsweise
- Strukturanten wie Glucose und Maleinsäure,
- haarkonditionierende Verbindungen wie Phospholipide, beispielsweise Sojalecithin, Ei-Lecitin und Kephaline, sowie Silikonöle,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Antischuppenwirkstoffe wie Piroctone Olamine und Zink Omadine,
- weitere Substanzen zur Einstellung des pH-Wertes,
- Wirkstoffe wie Panthenol, Pantothensäure, Allantoin, Pyrrolidoncarbonsäuren und deren Salze, Pflanzenextrakte und Vitamine,
- Cholesterin,
- Lichtschutzmittel,
- Konsistenzgeber wie Zuckerester, Polyolester oder Polyolalkylether,
- Fette und Wachse wie Walrat, Bienenwachs, Montanwachs, Paraffine, Fettalkohole und Fettsäureester,
- Fettsäurealkanolamide,
- Komplexbildner wie EDTA, NTA und Phosphonsäuren,
- Quell- und Penetrationsstoffe wie Propylenglykolmonoethylether, Carbonate, Hydrogencarbonate, Guanidine, Harnstoffe sowie primäre, sekundäre und tertiäre Phosphate, Imidazole, Tannine, Pyrrol,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft sowie
- Antioxidantien.

Ein zweiter Gegenstand der Erfindung ist ein Verfahren zum Färben von keratinhaltigen Fasern, insbesondere menschlichen Haaren, in welchem ein Mittel des ersten Gegenstandes der Erfindung auf das Haar aufgebracht wird, und nach einer Einwirkzeit wieder vom Haar abgespült wird.

Die erfindungsgemäßen Komponenten A, B und gegebenenfalls C können als farbgebende Komponenten entweder gleichzeitig auf das Haar aufgebracht werden oder aber auch nacheinander, d. h. in einem mehrstufigen Verfahren, wobei es unerheblich ist, welche der Komponenten zuerst aufgetragen wird. In der Regel werden die Komponenten gemeinsam mit einem wasserhaltigen, kosmetischen Träger in einer Menge von 100 g auf das Haar aufgebracht, ca. 30 Minuten dort belassen und anschließend ausgespült oder mit einem handelsüblichen Haarshampoo ausgewaschen.

In einer bevorzugten Ausführungsform des erfindungsgemäßen Verfahrens wird das erfindungsgemäße Färbemittel unmittelbar vor der Anwendung aus einer ersten Zusammensetzung, welche die Komponente A enthält, mit einer zweiten Zusammensetzung, enthaltend Komponenten B und gegebenenfalls C, gemischt.

Es ist bevorzugt, dass die erste Zusammensetzung einen pH-Wert von 1 bis 7, bevorzugt von 3 bis 6, besitzt und die zweite Zusammensetzung auf einen pH-Wert von 2 bis 11 eingestellt ist. Der pH-Wert der gebrauchsfertigen Anwendungsmischungen liegt üblicherweise zwischen 2 und 11, vorzugsweise zwischen 6 und 10. Wenn beide Zusammensetzungen flüssig sind, ist das Mischverhalten optimal, wenn die erste Zusammensetzung eine Viskosität von 50 bis 100000 mPa˙s besitzt und die zweite Zusammensetzung eine Viskosität von 15000 bis 150000 mPa˙s hat. Die Anwendungsmischung erhält eine Viskosität von 5000 bis 50000 mPa˙s, lässt sich leicht auftragen und erlaubt eine rasche Penetration der farbstoffbildenden Komponenten in das Haar. Alle Viskositäten werden bei 20°C mit einem Rotationsviskosimeter mit Spindel Nr. 4 bestimmt.

Die fakultativ enthaltenen Ammonium- oder Metallsalze können allein oder im Gemisch mit den weiteren Komponenten zugesetzt werden. Auch eine Vorbehandlung der Fasern mit der Salzlösung ist möglich.

Enthält das Haarfärbemittel neben den Komponenten A, B und gegebenenfalls C zusätzlich als chemisches Oxidationsmittel Wasserstoffperoxid oder ein wasserstoffperoxidhaltiges Oxidationsmittelgemisch, gegebenenfalls in Kombination mit anorganischen Peroxoverbindungen wie Persulfaten oder Peroxodisulfaten, so liegt der pH-Wert des wasserstoffperoxidhaltigen Haarfärbemittels vorzugsweise in einem pH-Bereich von pH 7 bis pH 11, besonders bevorzugt pH 8 bis pH 10. Das Oxidationsmittel kann unmittelbar vor der Anwendung mit dem Haarfärbemittel gemischt und die Mischung auf das Haar aufgebracht werden. Werden die Verbindungen der Komponente A und die Verbindungen der Komponenten B und gegebenenfalls C in einem zweistufigen Verfahren nacheinander auf das Haar appliziert, ist das Oxidationsmittel in einer der beiden Verfahrensstufen zusammen mit der entsprechenden farbgebenden Komponente anzuwenden. Zu diesem Zweck kann das Oxidationsmittel mit der Komponente A in einem Container oder getrennt konfektioniert werden.

Die erfindungsgemäßen Komponenten A, B und gegebenenfalls C können entweder getrennt oder zusammen gelagert werden, entweder in einer flüssigen bis pastösen Zubereitung (wässrig oder wasserfrei) oder als trockenes Pulver. Werden die Komponenten in einer flüssigen Zubereitung zusammen gelagert, so sollte diese zur Verminderung einer Reaktion der Komponenten weitgehend wasserfrei sein. Bei der getrennten Lagerung werden die reaktiven Komponenten erst unmittelbar vor der Anwendung miteinander innig vermischt. Bei der trockenen Lagerung wird vor der Anwendung üblicherweise eine definierte Menge warmen (30°C bis 80°C) Wassers hinzugefügt und eine homogene Mischung hergestellt.

### Beispiele

Alle Mengenangeben sind, soweit nicht anders gekennzeichnet, Gewichtsprozent (Gew.-%). Folgende Handelsprodukte wurden als Rohstoffe verwendet:
- Natrosol^{®} 250HR: Hydroxyethylcellulose (INCI-Bezeichnung: Hydroxyethylcellulose) (Hercules)

Es wurden folgende Komponenten nach bekanntem Verfahren hergestellt:

**Tabelle 1: Farbgele**

| | Gel 1 | Gel 2 |
|---|---|---|
| 4-Formyl-1-methylchinolinium p-Toluolsulfonat | 3,43 | - |
| (4-Amino-3-methylphenyl)(3-(imidazol-1-yl)propyl)amin | - | 3,50 |
| Ethylenglykol | 15,00 | - |
| 2-Phenoxyethanol | 0,80 | - |
| Natriumsalicylat | 0,40 | - |
| Natrosol^{®} 250 HR | 1,25 | 1,50 |
| Monoethanolamin | - | ad pH 10 |
| Weinsäurelösung, gesättigt | 0,80 | - |
| Parfum | 0,10 | - |
| Wasser, dest. | ad 100 | ad 100 |
| pH-Wert | 4,75 | 10,0 |

Die Gelkomponenten 1 und 2 der Tabelle 1 werden im Gewichtsverhältnis 1:1 zu einem erfindungsgemäßen Färbemittel gemischt und auf Wolläppchen sowie auf Haarsträhnen (Kerling naturweiß) appliziert (2g Farbmischung pro 1 g Haar bzw. Wolläppchen). Nach einer Einwirkzeit von 30 min bei 32°C wird die Farbmischung ausgespült, mit Shampoo gewaschen und das Haar bzw. Wolläppchen getrocknet. Es wurde eine intensive und gleichmäßige blauviolett Färbung mit guten Echtheitseigenschaften erhalten.

Es werden die gleichen Ergebnisse erzielt, wenn das Gel 1 der Tabelle 1 statt 4-Formyl-1-methylchinolinium p-Toluolsulfonat eine gleiche Menge 2-Formyl-1-methylchinolinium p-Toluolsulfonat enthält.

## Patentansprüche

1. Mittel zum Färben keratinhaltiger Fasern, insbesondere menschlicher Haare, welches eine Kombination aus Komponente
(A) mindestens einer Verbindung der Formel I oder deren Derivaten 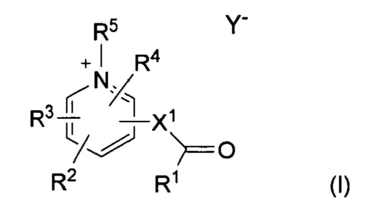 worin
- R¹ für ein Wasserstoffatom, eine (C₁-C₆)-Alkylgruppe, eine Arylgruppe oder eine Heteroarylgruppe steht,
- R², R³ und R⁴ jeweils unabhängig voneinander ein Wasserstoffatom, ein Halogenatom, eine (C₁-C₆)-Alkylgruppe, (C₁-C₆)-Alkoxygruppe, (C₁-C₆)-Hydroxylalkylgruppe, Hydroxy-(C₁-C₆)-alkoxygruppe, Hydroxygruppe, Nitrogruppe, Arylgruppe, Trifluormethylgruppe, Aminogruppe, die durch (C₁-C₆)-Alkylgruppen substituiert sein kann, oder (C₁-C₆)-Acylgruppe bedeuten, wobei auch zwei der Reste gemeinsam einen ankondensierten Benzolring bilden können,
- R⁵ für eine (C₁-C₆)-Alkylgruppe, Arylgruppe, Aryl-(C₁-C₆)-alkylgruppe oder Heteroarylgruppe steht,
- X¹ für eine direkte Bindung oder eine ggf. substituierte Vinylen- oder Phenylengruppe steht, und
- Y⁻ ein physiologisch verträgliches Anion bedeutet,
und Komponente
(B) mindestens einer Verbindung gemäß Formel (II), 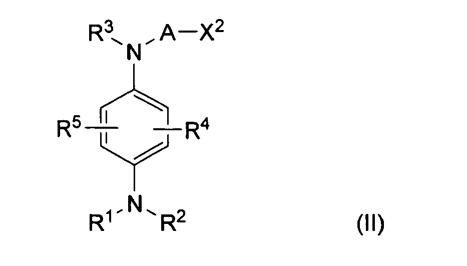
worin
- A steht für eine verzweigte oder unverzweigte Alkylengruppe mit 1 bis 6 Kohlenstoffatomen, die gegebenenfalls einen oder mehrere Substituenten, ausgewählt aus Hydroxygruppe(n) und Halogenatom(en), tragen kann,
- X² steht für einen gegebenenfalls substituierten Imidazolylrest,
- R¹, R² und R³ stehen unabhängig voneinander für ein Wasserstoffatom, eine C₁- bis C₄-Alkylgruppe, eine C₁- bis C₄-Monohydroxyalkylgruppe oder eine C₂- bis C₆- Polyhydroxyalkylgruppe, und
- R⁴ und R⁵ stehen unabhängig voneinander für ein Wasserstoffatom, eine C₁-bis C₄-Alkylgruppe, eine C₁- bis C₄-Alkoxygruppe, eine C₁- bis C₄-Monohydroxyalkylgruppe, eine C₂- bis C₄- Polyhydroxyalkylgruppe oder ein Halogenatom,
enthält.

2. Mittel nach Anspruch 1, **dadurch gekennzeichnet, daß** die Verbindungen der Formel (I) in einer Menge von 0,03 bis 65 mmol, insbesondere 1 bis 40 mmol, bezogen auf 100 g des anwendungsbereiten Mittels, enthalten sind.

3. Mittel nach einem der Ansprüche 1 oder 2, **dadurch gekennzeichnet, daß** die Verbindungen der Formel (I) aus Verbindungen der Formel (III) ausgewählt werden, 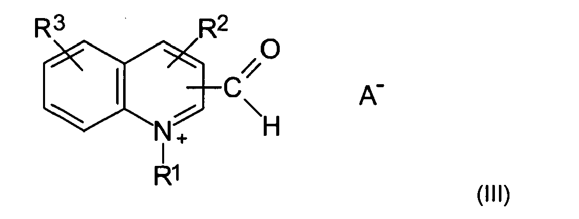 worin
R¹ für eine (C₁-C₆)-Alkylgruppe, (C₂-C₆)-Alkenylgruppe, Arylgruppe, Aryl-(C₁-C₆)-alkylgruppe oder Heteroaryl-(C₁-C₆)-alkylgruppe steht,
R² und R³ jeweils unabhängig voneinander ein Wasserstoffatom, ein Halogenatom, eine (C₁-C₆)-Alkylgruppe, eine (C₁-C₆)-Alkoxygruppe, Hydroxy-(C₁-C₆)-alkoxygruppe, Hydroxygruppe, Nitrogruppe, Arylgruppe, Trifluormethylgruppe, Aminogruppe, die durch (C₁-C₆)-Alkylgruppen substituiert sein kann, oder (C₁-C₆)-Acylgruppe bedeuten und
A⁻ ein physiologisch verträgliches Anion bedeutet.

4. Mittel nach einem der Ansprüche 1 bis 3, **dadurch gekennzeichnet, dass** die Verbindungen der Formel (I) aus der Gruppe bestehend aus den Benzolsulfonaten, p-Toluolsulfonaten, Methansulfonaten, Trifluormethansulfonaten, Perchloraten, Sulfaten, Chloriden, Bromiden, Jodiden und Tetrachlorzinkaten von 4-Formyl-1-methylchinolinium und 2-Formyl-1-methylchinolinium, ausgewählt werden.

5. Mittel nach einem der Ansprüche 1 bis 4, **dadurch gekennzeichnet, dass** die Gruppe X² gemäß Formel (II) für eine Gruppe der Formel (IVa) oder (IVb) steht 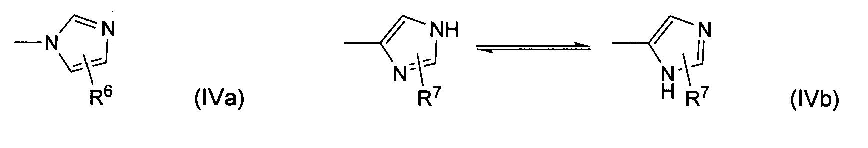 wobei R⁶ und R⁷ stehen unabhängig voneinander für ein Wasserstoffatom, eine C₁-bis C₄-Alkylgruppe, eine C₁- bis C₄-Monohydroxyalkylgruppe, eine C₂- bis C₄-Polyhydroxyalkylgruppe oder ein Halogenatom.

6. Mittel nach einem der Ansprüche 1 bis 5, **dadurch gekennzeichnet, dass** die Verbindungen der Formel (II) der Gruppe ausgewählt werden, die gebildet wird, aus (4-Aminophenyl)(3-(imidazol-1-yl)propyl)amin, (4-Amino-3-methylphenyl)(3-(imidazol-1-yl)propyl)amin und (4-Aminophenyl)(2-(imidazol-5-yl)ethyl)amin sowie den Salzen dieser Verbindungen.

7. Mittel nach einem der Ansprüche 1 bis 6, **dadurch gekennzeichnet, dass** die Verbindungen der Formel (II) in einer Menge von 0,03 bis 65 mmol, insbesondere 1 bis 40 mmol, bezogen auf 100 g des anwendungsbereiten Mittels, enthalten sind.

8. Mittel nach einem der Ansprüche 1 bis 7, **dadurch gekennzeichnet, dass** als Komponente C zusätzlich mindestens eine Verbindung enthalten ist, ausgewählt aus CH-aciden Verbindungen und/oder Verbindungen mit primärer oder sekundärer Aminogruppe, ausgewählt aus der Gruppe, die gebildet wird aus primären oder sekundären aromatischen Aminen, stickstoffhaltigen heterocyclischen Verbindungen und aromatischen Hydroxyverbindungen.

9. Mittel nach einem der Ansprüche 1 bis 8, **dadurch gekennzeichnet, dass** es zusätzlich mindestens ein lineares oder verzweigtes, aliphatisches Polyol enthält.

10. Mittel nach einem der Ansprüche 1 bis 9, **dadurch gekennzeichnet, dass** es zusätzlich mindestens ein Tensid enthält.

11. Verfahren zum Färben von keratinhaltigen Fasern, insbesondere menschlichen Haaren, in welchem ein Mittel nach einem der Ansprüche 1 bis 10 auf das Haar aufgebracht wird, und nach einer Einwirkzeit wieder vom Haar abgespült wird.
